# EUROPÄISCHE PATENTSCHRIFT

(11) **EP 3 723 868 B1**
(45) Veröffentlichungstag und Bekanntmachung des Hinweises auf die Patenterteilung: **15.01.2025**
(21) Anmeldenummer: 18811736.0
(22) Anmeldetag: 19.11.2018
(51) Int. Cl.: A62B 7/00, A61M 16/08, B63C 11/24, A61M 16/00, A61H 31/00, A61M 16/22

(54) **FEDERBRÜCKE FÜR EIN FEDERBRÜCKEN-ATEMBEUTELPLATTE-SYSTEM EINES KREISLAUFATEMSCHUTZGERÄTS, FEDERBRÜCKEN-ATEMBEUTELPLATTE-SYSTEM SOWIE KREISLAUFATEMSCHUTZGERÄTS**
SPRING LINK FOR A SPRING LINK BREATHING BAG PLATE SYSTEM OF A CLOSED-CIRCUIT BREATHING APPARATUS, SPRING LINK BREATHING BAG PLATE SYSTEM AND CLOSED-CIRCUIT BREATHING APPARATUS
LATTE FLEXIBLE POUR UN SYSTÈME DE PLAQUE DE SAC RESPIRATOIRE ET DE LATTES FLEXIBLES D'UN CIRCUIT D'APPAREIL RESPIRATOIRE, SYSTÈME DE PLAQUE DE SAC RESPIRATOIRE ET DE LATTES FLEXIBLES AINSI QUE CIRCUIT D'APPAREIL RESPIRATOIRE

(30) Priorität: 14.12.2017 DE 102017011581
(43) Veröffentlichungstag der Anmeldung: 21.10.2020
(73) Patentinhaber: Dräger Safety AG & Co. KGaA, 23560 Lübeck (DE)
(72) Erfinder: WILHELM, Christian, 23560 Lübeck (DE); DÜPJAN, Matthias, 23562 Lübeck (DE); KIRMSE, Sören, 23554 Lübeck (DE)
(74) Vertreter: Guthöhrlein, Gerhard
(86) Internationale Anmeldenummer: PCT/EP2018/081703
(87) Internationale Veröffentlichungsnummer: WO 2019/115159

(56) Entgegenhaltungen:
- WO-A2-2012/073024
- DE-A1- 102014 017 955
- FR-A- 1 076 247
- FR-A- 581 026

## Beschreibung

Die vorliegende Erfindung betrifft eine Federbrücke für ein Federbrücken-Atembeutelplatte-System eines Kreislaufatemschutzgeräts, aufweisend einen Federbrückenträger zur Anordnung wenigstens eines Federelements eines Federbrücken-Atembeutelplatte-Systems sowie aufweisend zumindest ein Befestigungselement zum Halten der Federbrücke am Kreislaufatemschutzgerät. Ferner betrifft die Erfindung ein Federbrücken-Atembeutelplatte-System für ein Kreislaufatemschutzgerät, aufweisend eine Atembeutelplatte, eine Federbrücke sowie wenigstens ein Federelement, welches zwischen der Atembeutelplatte und der Federbrücke angeordnet ist. Des Weiteren betrifft die Erfindung ein Kreislaufatemschutzgerät, aufweisend ein Gehäuse mit einem Gehäuseunterteil und einem Gehäuseoberteil, einen CO₂-Absorber, eine Sauerstoffflasche sowie ein Federbrücken-Atembeutelplatte-System.

### STAND DER TECHNIK

In Kreislaufatemschutzgeräten oder Beatmungssystemen für Notfall- oder Langzeitbeatmung von Patienten befinden sich in der Regel Atembeutel, auch Gegenlungen genannt, verschiedenster Ausführungen und Wirkweisen. Diese Gegenlungen bzw. Atembeutel sind flexible Volumina, welche das Atmen unter erschwerten Bedingungen oder in schädlicher Umgebung erleichtern.

Allen Atembeuteln gemein ist das Prinzip des Aufnehmens einer Gasmenge durch Entfaltung oder Aufblähen und das Abgeben einer Gasmenge durch Einfalten oder Kollabieren der gasumgebenen Hülle. Dabei kann das periodische Bewegen aktiv beispielsweise durch Hilfsaggregate oder passiv durch Volumeneinspeisung und Volumenentnahme erfolgen.

Solche Hilfsaggregate können zum Beispiel Federsysteme verschiedenster Ausführung sein. Dabei können Druck- oder Zugfedern verwendet werden. In jedem Fall wird durch das entsprechende Federsystem das Kollabieren oder Einfalten des Atembeutels unterstützt. Dabei bildet der Atembeutel in Verbindung mit einer geeigneten Atembeutelplatte das erste mechanische Gegenlager des Federsystems. Das zweite Gegenlager, auch als Federsitz bezeichnet, ist in der Regel eine u-förmige feste Komponente im Kreislaufatemschutzgerät, oft als Federbrücke bezeichnet. Diese Federbrücke übernimmt die Abstützung im Falle der Verwendung von Druckfedern oder die Aufhängung im Falle der Verwendung von Zugfedern. Die auf die Atembeutelplatte und damit auf die Atembeutelfläche wirkende Federkraft erzeugt im Inneren der gasumgebenden Hülle, also im Inneren des Atembeutels und des gesamtem Atemkreislaufsystems einen konstanten Überdruck. Dieser Überdruck fördert und erleichtert zum einen das Einatmen, also die Inhalation des Atemgases, und schützt gleichzeitig den Benutzer des Atemkreislaufsystems vor dem Eindringen von gesundheitsschädlichen Stoffen infolge der Druckrichtung.

Der Atembeutel wird auch als Auffangvolumen oder aktives Gasreservoir für die vom Kohlenstoffdioxid gereinigte Ausatemluft benötigt. Aufgrund der Tatsache, dass der Atembeutel im Inneren Kontakt zum Atemgas und auf der Außenseite zur Umgebung hat, häufig toxisch, ist regelmäßige Reinigung und Desinfektion des Atembeutels notwendig. Somit ist es wichtig und erforderlich den Atembeutel nach jeder Benutzung des Atemkreislaufsystems zu reinigen und zu desinfizieren. Dazu müssen in der Regel die korrespondierenden Bauteile oder Komponenten in der Peripherie des Atembeutels entfernt werden. Auch das entsprechende Federsystem, je nachdem in welcher Art und Weise dieses mit dem Atembeutel verbunden ist, muss entfernt werden. Zusätzlich haben die meisten Kreislaufatemschutzgeräte ein mechanisches Führungssystem und/oder pneumatische Verbindungselemente, welche ebenfalls für den Reinigungs- und Desinfektionsprozess demontiert werden müssen. Aufgrund der typischerweise geringen Platzverhältnisse innerhalb der Kreislaufatemschutzgeräte ist die Demontage und Montage des Atemkreislaufes sehr aufwendig und für ungeübte Anwender sehr zeitintensiv und schwierig.

Ausgangslage für die Erfindung ist die Atembeutel-Federbrücken-Ausführung des Kreislaufatemschutzgeräts der Anmelderin, welches im Wesentlichen für das Grubenrettungswesen, aber auch im Bereich der Feuerwehr, insbesondere bei Langzeiteinsätzen, verwendet wird. Bei diesem Kreislaufatemschutzgerät befinden sich auf dem oberen Teil den Atembeutels Aufnahmepunkte zum Befestigen einer Atembeutelplatte, welche als Gegenlager und Kraftverteilung von korrespondierenden Druckfedern für den inneren Überdruck dienen.

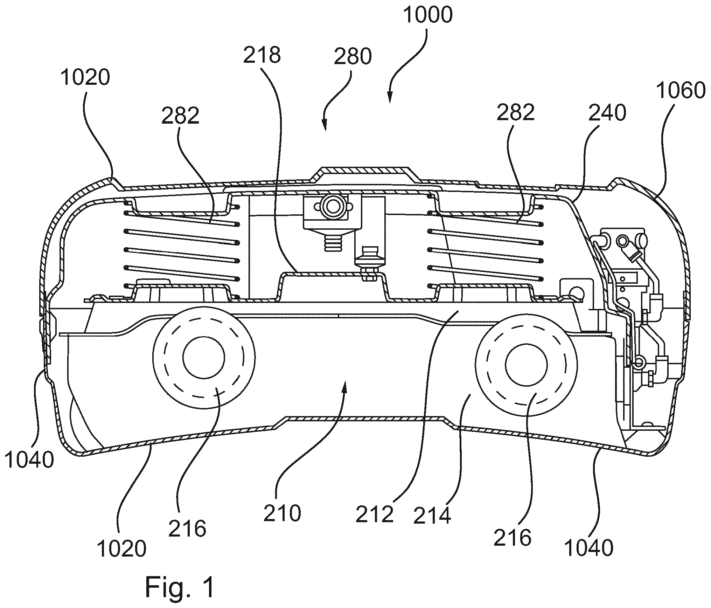

In Fig. 1 ist schematisch ein Federbrücken-Atembeutelplatte-System (80) des Kreislaufatemschutzgerätes (1000) der Anmelderin in einer Schnittdarstellung dargestellt. Das Kreislaufatemschutzgerät (1000) weist ein Gehäuse (1020) mit einem Gehäuseunterteil (1040) und einem Gehäuseoberteil (1060) auf. Der Atembeutel (210) weist eine kleinere Oberseite (212) und eine größere Unterseite (214) auf. Ebenso dargestellt sind die Anschlüsse, Muffen (216) zum Verbinden des Atembeutels (210) mit einem Kohlenstoffdioxid-Absorber und einer Atemgaskühler-Einheit.

Die Anschluss-Muffen (216) müssen zum Verbinden mit den anderen Komponenten von Hand stark gedehnt werden, damit ein entsprechender Hinterschnitt in der Muffe (216) in eine entsprechende Nutgeometrie im korrespondierenden Bauteil seinen Sitz findet. Auf der Oberseite (212) des Atembeutels (210) befinden sich Aufnahmepunkte für eine Druckplatte (218), die sogenannte Atembeutelplatte (218), welche wiederum als Gegenlager und Sitz für die Druckfedern (282) dient. Die Druckfedern (282) erzeugen im Zusammenspiel mit der Atembeutelplatte (218) aufgrund der Fläche und Kraft einen steten Überdruck im Atembeutel (210). Dieser verhindert aufgrund der Druckrichtung das Eindringen von Schadstoffen und schützt somit den Geräteträger bei der Atmung. Das zweite Gegenlager für die beiden Druckfedern (282) bildet die sogenannte Federbrücke (240). Diese ist in diesem Kreislaufatemschutzgerät (1000) fest, d.h. nicht lösbar, mit dem Gehäuseunterteil (1040) verbunden. Einbauposition und Peripherie sind in der Fig. 1 dargestellt. Zur Demontage des Atembeutels (210) bspw. nach dem Einsatz des Kreislaufatemschutzgeräts (1000) als Vorbereitung der Reinigung und Desinfektion müssen die Druckfedern (282) entfernt werden. Dies geschieht durch Zusammendrücken der Druckfedern (282).

Nachteile dieses Kreislaufatemschutzgeräts (1000) sind, dass im Laufe der Produktlebenszeit es immer wieder verschiedene Defekte an verschiedenen Komponenten des Atemkreislaufes, insbesondere am Atembeutel (210) gib. Dies ist zum einen der technischen Auslegung und zum anderen der Einbauposition des Atembeutels (210) im Kreislaufatemschutzgerät (1000) sowie der Notwendigkeit des Ein- und Ausbaus des Atembeutels (210) aus dem Kreislaufatemschutzgerät (1000) zur Reinigung und Desinfektion zuzuschreiben. Sehr häufig wird während der Montage und Demontage des Atembeutels (210) ein am Atembeutel (210) befindliches Minimumventil (nicht dargestellt) beschädigt. In der Regel ist ein deformierter Ventilhebel Resultat der Schwierigkeiten beim Ein- und Ausbau des Atembeutels (210). Auch der Atembeutel (210) selbst kann leicht beschädigt werden. Zurückzuführen ist dies unter anderem auf die beengten Platzverhältnisse und Konnektierungs-Prinzipien. Da die Federbrücke (240) ein fest installiertes Bauteil am unteren Gerätegehäuse (1020) ist, ermöglicht sie kein barrierefreies Arbeiten innerhalb des Kreislaufatemschutzgerätes (1000). Um die Druckfedern (282) zwischen Atembeutel (210) und Federbrücke (240) erreichen zu können, müssen einige, vorzugsweise alle anderen Komponenten zuvor aus dem Geräteinnenraum demontiert werden, da die Bewegungsräume für die Hände eines Monteurs sehr eingeschränkt sind. Nachdem ein Absorber und ein Atemluftkühler des Kreislaufatemschutzgerätes (1000) entnommen sind, kann die Entnahme des Atembeutels (210) durch den Ausbau des Minimumventils erfolgen. Ein weiterer Nachteil ist die Notwendigkeit des Demontierens der Atembeutelführung. Dieser Metallhebel ist an einem Ende direkt mit der Atembeutelplatte (218) mittels eines Druckknopfs befestigt und auf der gegenüberliegenden Seite außerhalb des Federbrücken-Atembeutelplatte-Systems beweglich montiert.

Sehr häufig ist es notwendig aufgrund von umfangreichen Einsatzszenarien viele Kreislaufatemschutzgeräte zu demontieren, zu reinigen und anschließend wieder zu montieren und zu prüfen. Dies kommt in der Regel bei großen Feuerwehreinsätzen mit viel Personal für den Einsatz von Kreislaufatemschutzgeräten vor. Aufgrund der Gefahrenlage und der begrenzten Anzahl an Kreislaufatemschutzgeräten besteht sehr oft die Notwendigkeit gebrauchte Kreislaufatemschutzgeräte schnellstens wieder für den erneuten Einsatz vorzubereiten. Dies ist bei dem zuvor beschriebenen Kreislaufatemschutzgerät der Anmelderin lediglich durch sehr gut ausgebildetes und trainiertes Personal zu leisten. Sämtliche Handgriffe sind nicht mit solchen aus dem täglichen Leben zu vergleichen. Somit ist eine intuitive Benutzung und Bedienung nahezu ausgeschlossen.

Die WO2012/073024 A2 offenbart eine Federbrücke, die lösbar an einem Gehäuseabschnitt eines Atemgerätes befestigt ist.

Ausgehend von diesem Stand der Technik hat der Erfindung die Aufgabe zugrunde gelegen, diese Nachteile von Federbrücken-Atembeutelplatte-Systemen eines Kreislaufatemschutzgeräts und damit eines Kreislaufatemschutzgeräts zumindest teilweise zu beheben. Es ist daher die Aufgabe der vorliegenden Erfindung, eine Federbrücke für ein Federbrücken-Atembeutelplatte-System eines Kreislaufatemschutzgeräts, ein Federbrücken-Atembeutelplatte-System für ein Kreislaufatemschutzgerät sowie ein Kreislaufatemschutzgerät bereitzustellen, welche auf eine einfache und schnelle Art und Weise die Montage und Demontage eines Atembeutels eines Kreislaufatemschutzgeräts zu Reinigungszwecken ermöglichen. Insbesondere soll innerhalb des Kreislaufatemschutzgeräts im Bereich des Federbrücken-Atembeutelplatte-Systems eine reduzierte Anzahl an Einzelteilen und mehr Platz für Wartungsprozeduren geschaffen werden.

### OFFENBARUNG DER ERFINDUNG

Voranstehende Aufgabe wird gelöst durch eine Federbrücke für ein Federbrücken-Atembeutelplatte-System eines Kreislaufatemschutzgeräts mit den Merkmalen des Anspruchs 1, durch ein Federbrücken-Atembeutelplatte-System für ein Kreislaufatemschutzgerät mit den Merkmalen des Anspruchs 9 sowie durch ein Kreislaufatemschutzgerät mit den Merkmalen des Anspruchs 13. Weitere Merkmale und Details der Erfindung ergeben sich aus den Unteransprüchen, der Beschreibung und den Zeichnungen. Dabei gelten Merkmale und Details, die im Zusammenhang mit dem erfindungsgemäßen Federbrücke beschrieben sind, gelten selbstverständlich auch im Zusammenhang mit dem erfindungsgemäßen Federbrücken-Atembeutelplatte-System sowie dem erfindungsgemäßen Kreislaufatemschutzgerät, und jeweils umgekehrt, sodass bezüglich der Offenbarung zu den einzelnen Erfindungsaspekten stets wechselseitig Bezug genommen wird bzw. werden kann.

Gemäß dem vorliegenden Aspekt der Erfindung wird die Aufgabe gelöst durch eine Federbrücke für einen Atembeutel eines Kreislaufatemschutzgeräts, aufweisend einen Federbrückenträger zur Anordnung wenigstens eines Federelements eines Federbrücken-Atembeutelplatte-Systems sowie aufweisend zumindest ein Befestigungselement zum Halten der Federbrücke am Kreislaufatemschutzgerät. Ferner ist das zumindest eine Befestigungselement ein Drehelement und/oder Schiebeelement zum dreh- und/oder schiebbeweglichen Bewegen der Federbrücke am und relativ zum Kreislaufatemschutzgerät, wobei die Federbrücke mittels dem Drehelement und/oder dem Schiebeelement aus einer Arbeitsposition in eine Öffnungsposition drehbar und/oder verschiebbar ist zum Freigeben eines Zugangs zu einem Atembeutel des Kreislaufatemschutzgeräts.

Eine derartig ausgebildete Federbrücke eines Federbrücken-Atembeutelplatte-Systems für ein Kreislaufatemschutzgerät ermöglicht eine einfache und schnelle Montage sowie Demontage eines unterhalb der Federbrücke angeordneten Atembeutels eines Kreislaufatemschutzgeräts. Die an einem Kreislaufatemschutzgerät gehaltene Federbrücke kann aufgrund der speziellen Ausgestaltung des zumindest einen Befestigungselementes als Drehelement und/oder als Schiebeelement sehr einfach aus einer Arbeitsposition in eine Öffnungsposition gedreht und/oder verschoben werden, um so den Zugang zu dem Atembeutel freizugeben. In der Arbeitsposition dient die Federbrücke als Gegenlager für Federelemente, die zwischen der Federbrücke und dem Atembeutel angeordnet sind. D. h., in der Arbeitsposition dient die Federbrücke als Abstützung der Druckfedern beziehungsweise Zugfedern. In der Arbeitsposition ist die Federbrücke unbeweglich, vorzugsweise verrastet, am Kreislaufatemschutzgerät angeordnet.

Vorzugsweise ist die Federbrücke in der Arbeitsposition parallel zu der Oberseite des Atembeutels innerhalb des Kreislaufatemschutzgeräts angeordnet. Hierdurch können die an der Federbrücke abgestützten Federelemente des Federbrücken-Atembeutelplatte-Systems eine Federkraft auf den Atembeutel ausüben. Aufgrund der Ausgestaltung des Befestigungselementes als Drehelement und/oder Schiebeelement kann die Federbrücke aus der Arbeitsposition durch eine Dreh- und/oder i Schiebebewegung n eine sogenannte Öffnungsposition überführt werden. D. h., vorzugsweise kann die Federbrücke mittels des Drehelementes relativ zum Kreislaufatemschutzgerät bzw. zum Gehäuse des Kreislaufatemschutzgeräts gedreht bzw. verschwenkt werden, um so den Zugang zu dem in der Arbeitsposition unterhalb der Federbrücke angeordneten Atembeutel freizugeben.

Alternativ oder zusätzlich zu der Drehbewegung kann die Federbrücke mittels des zumindest einen Schiebeelementes aus der Arbeitsposition in eine Öffnungsposition verschoben werden, um so den Zugang zu dem unterhalb der Federbrücke angeordneten Atembeutel freizugeben. Durch die Verdrehbarkeit bzw. Verschiebbarkeit der Federbrücke relativ zu einem Kreislaufatemschutzgerät bzw. zum Gehäuse eines Kreislaufatemschutzgerätes, kann ein Monteur einfach und schnell zu Reinigungszwecken oder Demontagezwecken zu dem Atembeutel des Kreislaufatemschutzgerätes gelangen.

Dadurch, dass das zumindest eine Befestigungselement als Drehelement und/oder Schiebeelement an der Federbrücke angeordnet ist, kann auf sehr einfache und schnelle Art und Weise die Federbrücke relativ zum Kreislaufatemschutzgerät verschwenkt bzw. verschoben werden, um so den Zugang zum Atembeutel freizugeben. Das zumindest eine Befestigungselement der Federbrücke ist vorzugsweise an einem Gegenbefestigungselement des Kreislaufatemschutzgerätes, insbesondere rotatorisch beweglich, angeordnet. Das Gegenbefestigungselement ist vorzugsweise an einer Innenwandung eines Gehäuses, insbesondere einer Geräteunterschale bzw. einem Geräteunterteil, des Kreislaufatemschutzgeräts dreh- und/oder verschiebbeweglich gelagert.

Die Federbrücke ist vorzugsweise als U-förmiges Profil ausgebildet, wobei der Federbrückenträger den Boden des U-förmig ausgebildeten Profils bildet und an den freien Enden der beiden Schenkel des U-förmigen Profils jeweils ein Befestigungselement in Form eines Drehelementes und/oder Schiebeelementes angeordnet ist. Hierdurch ist eine besonders einfache Schwenkbarkeit bzw. Verschiebbarkeit der Federbrücke relativ zum Gehäuse eines Kreislaufatemschutzgeräts gewährleistet.

Eine derartig ausgebildete Federbrücke schafft aufgrund der Bewegbarkeit der Federbrücke aus einer Arbeitsposition in eine Öffnungsposition mehr Platz für Wartungsprozeduren innerhalb des Kreislaufatemschutzgerätes, insbesondere an einem Federbrücken-Atembeutelplatte-System des Kreislaufatemschutzgeräts beziehungsweise einem Atembeutel des Kreislaufatemschutzgerätes. Der Atembeutel unterhalb des Federbrücken-Atembeutelplatte-Systems ist in der Öffnungsposition der Federbrücke und damit des Federbrücken-Atembeutelplatte-Systems leicht zugänglich und kann in dieser Öffnungsposition sehr leicht zu Reinigungszwecken oder Austauschzwecken demontiert werden. Entsprechend einfach ist die Montage des gereinigten oder neuen Atembeutels in das Kreislaufatemschutzgerät, wenn die Federbrücke und damit des Federbrücken-Atembeutelplatte-System sich in der Öffnungsposition befinden.

Gemäß einer bevorzugten Weiterentwicklung der Erfindung kann bei einer Federbrücke vorgesehen sein, dass das Befestigungselement beziehungsweise das Drehelement ein Drehgelenk, ein Kreuzgelenk, ein Winkelelement, ein Kugelgelenk, ein Scharnierelement oder eine Steckaufnahme zur Befestigung an einem Zapfen des Kreislaufatemschutzgeräts ist. Derartig ausgebildete Drehelemente ermöglichen ein besonders einfaches Verdrehen bzw. Verschwenken der Federbrücke relativ zum Gehäuse des Kreislaufatemschutzgerätes und damit eine einfache und schnelle Zugänglichkeit des in der Arbeitsposition unterhalb der Federbrücke angeordneten Atembeutels des Kreislaufatemschutzgeräts. Die Federbrücke kann derartig ausgebildet sein, dass diese lediglich ein einziges Befestigungselement, insbesondere ein einziges Drehelement, aufweist, mittels welchem die Federbrücke zwischen der Arbeitsposition und der Öffnungsposition hin- und herbewegt werden kann. Beispielsweise kann an dem Federbrückenträger der Federbrücke abgewinkelt ein Profil angeordnet sein, an dessen Ende das eine Befestigungselement angeordnet ist. Dieses eine Befestigungselement kann drehbeweglich an einem entsprechenden Gegenbefestigungselement am Gehäuse des Kreislaufatemschutzgerätes, beispielsweise an einem Zapfen, schwenkbeweglich angeordnet sein. Besonders bevorzugt weist eine Federbrücke jedoch zwei Befestigungselemente auf, mittels denen die Federbrücke an entsprechenden Gegenbefestigungselementen des Gehäuses eines Kreislaufatemschutzgerätes, insbesondere des Gehäuseunterteils eines Kreislaufatemschutzgerätes, schwenkbeweglich gelagert ist.

Bevorzugt kann ferner bei einer Federbrücke vorgesehen sein, dass das zumindest eine Befestigungselement an wenigstens einem Kragarm der Federbrücke angeordnet ist, wobei der wenigstens eine Kragarm abgewinkelt zum Federbrückenträger an dem Federbrückenträger angeordnet ist. Insbesondere vorteilhaft ist eine Federbrücke, bei der zwei Kragarme an zwei gegenüberliegenden Seiten des Federbrückenträgers abgewinkelt zum Federbrückenträger angeordnet sind. Insbesondere bilden die beiden Kragarme zusammen mit dem Federbrückenträger ein U-förmiges bzw. annähernd U-förmiges Profil. Vorzugsweise sind an den freien Enden der Kragarme jeweils Befestigungselemente, vorzugsweise Drehelemente, angeordnet, mittels derer die Federbrücke dreh- beziehungsweise schwenkbeweglich an entsprechenden Gegenbefestigungselementen des Gehäuses des Kreislaufatemschutzgerätes gelagert werden kann. Eine derartig ausgebildete Federbrücke kann beispielsweise einfach durch eine 90°-Drehung beziehungsweise ungefähr 90°-Drehung aus der Arbeitsposition in die Öffnungsposition gedreht bzw. verschwenkt werden, sodass in der Öffnungsposition die Federbrücke bzw. der Federbrückenträger der Federbrücke senkrecht bzw. annähernd senkrecht zur Gehäuseunterseite des Kreislaufatemschutzgeräts verläuft. Hierdurch ist ein Atembeutel, der in der Arbeitsposition von der Federbrücke verdeckt war, frei zugänglich.

Vorzugsweise sind an den dem Federbrückenträger abgewandten Ende der Kragarme Steckaufnahmen als Drehelemente vorgesehen, die mit entsprechend als Zapfen ausgebildeten Gegenbefestigungselementen der Gehäusewandung des Kreislaufatemschutzgerätes rotatorisch miteinander verbunden sind. Im Unterschied zu dem bekannten Federbrücken-Atembeutelplatte-System ermöglicht eine derartig ausgebildete Federbrücke ein Klappen der Federbrücke und sogar ein Entnehmen der Federbrücke aus dem Kreislaufatemschutzgerät. Für den Anwender entsteht somit der Vorteil des Zugewinns an Raum für alle weiteren Tätigkeiten, welche notwendig sind, um das Kreislaufatemschutzgerät zu warten. Die Federbrücke ist nicht mehr, wie bei dem bekannten Federbrücken-Atembeutelplatte-System an der Geräteunterschale dauerhaft fest angeordnet, insbesondere vernietet, sondern beweglich und/oder entfernbar an der Geräteunterschale gehalten. Insbesondere durch die Ausbildung der Drehelemente als Steckaufnahmen kann die Federbrücke sowohl um Zapfen, die die Gegenbefestigungselemente für die Steckaufnahmen bilden, gedreht werden, als auch von diesen unter Überwindung einer Klemmkraft einfach entfernt werden. Hierdurch ist eine besonders einfache Zugänglichkeit zu dem Atembeutel des Kreislaufatemschutzgeräts ermöglicht, damit dieser von einem Benutzer aus dem Kreislaufatemschutzgerät entfernt und entsprechend einer Reinigung zugeführt werden kann.

Gemäß einer bevorzugten Weiterbildung der Erfindung kann bei einer Federbrücke vorgesehen sein, dass das Schiebeelement eine Führungskulisse oder ein Führungszapfen zur Führung der Federbrücke an einem Gegenschiebeelement, insbesondere einem Gegenführungszapfen oder einer Gegenführungskulisse, des Kreislaufatemschutzgerätes ist. Dies ermöglicht ein Verschieben, insbesondere ein lineares Verschieben der Federbrücke relativ zum Kreislaufatemschutzgerät. Durch das Verschieben der Federbrücke aus der Arbeitsposition in eine Öffnungsposition oder eine Zwischenöffnungsposition kann zusätzlicher Raum für die Montage bzw. Demontage eines Atembeutels eines Kreislaufatemschutzgerätes geschaffen werden. Das mindestens eine Schiebeelement kann beispielsweise auch als Teleskopführung ausgebildet sein, durch welches die Federbrücke linear verschiebbar zwischen der Arbeitsposition und der Öffnungsposition oder einer Zwischenöffnungsposition verschoben werden kann. Vorzugsweise ist jeweils ein Schiebeelement an den zwei Kragarmen einer U-förmig ausgebildeten Federbrücke vorgesehen.

Gemäß einer besonders bevorzugten Weiterentwicklung der Erfindung kann bei einer Federbrücke vorgesehen sein, dass die Federbrücke wenigstens ein Halteelement zum Halten der Federbrücke in einer Arbeitsposition und zum Halten der Federbrücke in einer Öffnungsposition aufweist. Durch das oder die Halteelemente kann die Federbrücke in der jeweiligen Position relativ zum Gehäuse des Kreislaufatemschutzgerätes gehalten werden. D. h., in der Arbeitsposition hält das Halteelement die Federbrücke fest relativ zum Gehäuse des Kreislaufatemschutzgerätes, sodass die Federbrücke das Gegenlager für die Federelemente eines Federbrücken-Atembeutelplatte-Systems bildet. Das wenigstens eine Halteelement ist vorzugsweise als Rastelement oder Klemmelement ausgebildet. D. h., vorzugsweise ist das Halteelement bzw. sind die Halteelemente derart ausgebildet, dass in der Arbeitsposition durch Verrasten oder Verklemmen die Gerätebereitschaft dargestellt wird und durch ein Verrasten oder Verklemmen in der Öffnungsposition die Entnahme der Federbrücke zur Wartung erleichtert ermöglicht wird. In beiden Positionen kann die Federbrücke aufgrund des oder der Halteelemente(s) unbeweglich zum Kreislaufatemschutzgerät gehalten werden.

Besonders bevorzugt ist eine Federbrücke, bei der der Federbrückenträger eine Arretierungsvorrichtung zum Arretieren einer durch das wenigstens eine Federelement des Federbrücken-Atembeutelplatte-Systems federkraftbeaufschlagten Atembeutelplatte des Federbrücken-Atembeutelplatte-Systems an dem Federbrückenträger aufweist. D. h., die Arretierungsvorrichtung dient zur Fixierung der Atembeutelplatte eines Federbrücken-Atembeutelplatte-Systems an dem Federbrückenträger der Federbrücke. Eine derartige Arretierungsvorrichtung dient zur Optimierung des Ein- und Ausbaus der Federbrücke beziehungsweise des Federbrücken-Atembeutelplatte-Systems aus dem Kreislaufatemschutzgerät. Zur Demontage der Federbrücke bzw. des Federbrücken-Atembeutelplatte-Systems wird die Atembeutelplatte des Federbrücken-Atembeutelplatte-Systems, welche direkt, aber lösbar, auf dem Atembeutel angeordnet ist, in Richtung des Federbrückenträgers gezogen und mit der Arretierungsvorrichtung der Federbrücke arretiert, insbesondere verrastet oder verklemmt. Zum Arretieren der Atembeutelplatte an dem Federbrückenträger wird das wenigstens eine Federelement des Federbrücken-Atembeutelplatte-Systems, insbesondere die zwei Druckfedern, auf ein Minimum komprimiert, sodass eine sehr kompakte Einheit, aufweisend die Federbrücke, die Atembeutelplatte und das wenigstens eine Federelement, entsteht. Diese kompakte Einheit, die das Federbrücken-Atembeutelplatte-System grundlegend darstellt, kann aufgrund des wenigstens einen Drehelementes und/oder Schiebeelementes in einem einfachen Bewegungsablauf verdreht bzw. verschoben und gegebenenfalls aus dem Kreislaufatemschutzgerät entnommen werden. Somit ist durch einfache Art und Weise ein freier Zugang zum Atembeutel des Kreislaufatemschutzgerätes und allen weiteren Komponenten innerhalb des Kreislaufatemschutzgerätes gegeben. Der Atembeutel kann in der Öffnungsposition der Federbrücke einfach von dem Federbrücken-Atembeutelplatte-System, insbesondere von der Atembeutelplatte des Federbrücken-Atembeutelplatte-Systems, demontiert werden und entsprechend einer Reinigung zugeführt werden. Die Montage der Federbrücke und damit des Federbrücken-Atembeutelplatte-Systems an das Kreislaufatemschutzgerät erfolgt in umgekehrter Reihenfolge. Die Federbrücke bzw. kompakte Einheit des Federbrücken-Atembeutelplatte-Systems wird mittels des wenigstens einen Befestigungselementes an dem Gegenbefestigungselement der Gehäusewandung des Kreislaufatemschutzgerätes schwenk- und/oder schiebebeweglich befestigt und aus der Öffnungsposition in die Arbeitsposition geklappt beziehungsweise verschoben und dort mittels des Halteelementes arretiert. Nachdem die Federbrücke die Arbeitsposition erreicht hat und in dieser verrastet oder verklemmt ist, kann die Arretierungsvorrichtung gelöst werden, sodass die Atembeutelplatte des Federbrücken-Atembeutelplatte-Systems aufgrund der Federkraft des wenigstens einen Federelementes ihre Position am Atembeutel findet und eine entsprechend Federkraft auf den Atembeutel ausüben kann.

In einer bevorzugten Weiterentwicklung der Erfindung kann bei einer Federbrücke vorgesehen sein, dass die Arretierungsvorrichtung über eine Schwenkachse an dem Federbrückenträger schwenkbeweglich angelenkt ist, insbesondere derart, dass in einer Freigabeposition, in der die Atembeutelplatte des Federbrücken-Atembeutelplatte-Systems nicht mittels der Arretierungsvorrichtung an dem Federbrückenträger gehalten ist, die Arretierungsvorrichtung plan oder annähernd plan zu dem Federbrückenträger verläuft, und in einer Arretierposition, in der die Atembeutelplatte des Federbrücken-Atembeutelplatte-Systems mittels der Arretierungsvorrichtung an dem Federbrückenträger gehalten ist, die Arretierungsvorrichtung geneigt, insbesondere senkrecht, zu dem Federbrückenträger verläuft. Durch eine derartig ausgebildete Federbrücke ist bereits durch die Stellung der Arretierungsvorrichtung relativ zum Federbrückenträger für einen Nutzer erkennbar, ob die Atembeutelplatte des Federbrücken-Atembeutelplatte-Systems an der Federbrücke fixiert ist oder eben nicht. D. h., die Arretierungsvorrichtung, die vorzugsweise als Arretierungshebel ausgebildet ist, ist in Größe und Klapprichtung derart gestaltet, dass es bei aufgestellter Arretierungsvorrichtung, also dann, wenn die Atembeutelplatte arretiert und gehalten wird, es nicht möglich ist, den Gehäusedeckel, d. h. das Gehäuseoberteil eines Kreislaufatemschutzgerätes, zu verschließen. Der Benutzer wird dadurch zur Kontrolle des Atemkreislaufs des Kreislaufatemschutzgerätes gezwungen, was letztlich die Sicherheit des gesamten Kreislaufatemschutzgerätes erhöht. Eine Benutzung des Kreislaufatemschutzgerätes mit an dem Federbrückenträger arretierter Atembeutelplatte ist nicht möglich.

Die Arretierungsvorrichtung ist vorzugsweise als Rastelement oder Klemmelement ausgebildet. Auch ein Bajonettverschluss ist möglich. In der Freigabeposition der Arretierungsvorrichtung verläuft die Arretierungsvorrichtung vorzugsweise parallel bzw. eben zu dem Federbrückenträger der Federbrücke. In dieser Freigabeposition hintergreift die Arretierungsvorrichtung nicht die Atembeutelplatte, sodass diese frei beweglich zur Federbrücke angeordnet bleibt. In der Arretierposition hintergreift die Arretierungsvorrichtung die Atembeutelplatte und hält diese dadurch an dem Federbrückenträger der Federbrücke fest. Die Arretierungsvorrichtung ist um eine Schwenkachse an dem Federbrückenträger schwenkbeweglich gelagert, sodass diese einfach aus der Freigabeposition in eine Arretierposition und umgekehrt überführbar ist. Bevorzugt ist die Arretierungsvorrichtung zentral am beziehungsweise in dem Federbrückenträger der Federbrücke angeordnet. So kann die Atembeutelplatte zentriert an dem Federbrückenträger gehalten werden.

Ein Benutzer des Kreislaufatemschutzgerätes kann zu Reinigungszwecken manuell die Atembeutelplatte entgegen der Federkraft des wenigstens einen Federelementes des Federbrücken-Atembeutelplatte-Systems in Richtung des Federbrückenträgers der Federbrücke bewegen und dann durch Verschwenken der Arretierungsvorrichtung aus der Freigabeposition in die Arretierposition die Atembeutelplatte an dem Federbrückenträger arretieren. Die Arretierungsvorrichtung ist dabei derart ausgebildet, dass diese die Atembeutelplatte in der Arretierposition fest an der Federbrücke hält, sodass diese nicht aufgrund der Komprimierung des wenigstens einen Federelementes leicht in eine Ausgangsposition zurückspringen kann. Die so entstehende, sehr kompakte Einheit aus Federbrücke, Atembeutelplatte und wenigstens einem Federelement, kann besonders einfach verschwenkt und/oder relativ zum Gehäuse eines Kreislaufatemschutzgerätes verschoben werden, um so eine einfache Zugänglichkeit zu dem Atembeutel des Kreislaufatemschutzgerätes zu gewährleisten. Die insbesondere senkrechte Anordnung der Arretierungsvorrichtung bzw. des Arretierhebels zu dem Federbrückenträger in der Arretierposition stellt sicher, dass eine Gehäuseoberseite des Kreislaufatemschutzgerätes nicht an die Geräteunterseite des Kreislaufatemschutzgerätes montiert werden kann. Erst wenn die Arretierungsvorrichtung aus der Arretierposition in die Freigabeposition überführt wurde und somit die Bewegung der Atembeutelplatte wieder freigegeben wurde, kann das Gehäuseoberteil des Kreislaufatemschutzgerätes an dem Gehäuseunterteil des Kreislaufatemschutzgerätes montiert werden. Hierdurch ist eine Kontrolle der Funktionssicherheit des Federbrücken-Atembeutelplatte-Systems und damit des Kreislaufatemschutzgerätes sichergestellt. Der Benutzer wird durch die spezielle Anordnung und Funktionalität einer derartigen Arretierungsvorrichtung einer Federbrücke zur Kontrolle des Atemkreislaufes gezwungen, was letztlich die Sicherheit des gesamten Kreislaufatemschutzgerätes erhöht. Eine Benutzung des Kreislaufatemschutzgerätes mit arretierter Atembeutelplatte ist nicht möglich.

Ein Federbrücken-Atembeutel-System weist wenigstens ein Federelement auf, welches zwischen der Atembeutelplatte und der Federbrücke bzw. dem Federbrückenträger der Federbrücke angeordnet ist. Das wenigstens eine Federelement kann verschiedenartig ausgebildet sein. Besonders bevorzugt weist ein Federbrücken-Atembeutel-System zwei Druckfedern oder zwei Zugfedern auf. Um derartige Druck- bzw. Zugfedern an der Federbrücke sicher zu halten, ist eine Federbrücke bevorzugt, bei der der Federbrückenträger wenigstens ein erstes Aufnahmeelement zur Anordnung wenigstens eines ersten Endes des wenigstens einen Federelements des Federbrücken-Atembeutel-Systems aufweist. D. h., an der zu dem Atembeutel zugewandten Seite des Federbrückenträgers befindet sich eine sogenannte Aufnahmegeometrie für ein Ende der Druckfedern bzw. Zugfedern des Federbrücken-Atembeutel-Systems. Die Aufnahmegeometrie bzw. das erste Aufnahmeelement kann eine ringförmige Nut oder ein zylinderförmiger Vorsprung, beispielsweise ein hülsenförmigen oder topfförmiger Vorsprung, sein. Das wenigstens eine Aufnahmeelement dient als Gegenlager für das wenigstens eine Federelement und hält somit das wenigstens eine Federelement in einer definierten Position relativ zu dem Federbrückenträger der Federbrücke. Das wenigstens eine Federelement kann zusätzlich mittels Fixierelementen an dem ersten Aufnahmeelement des Federbrückenträgers gehalten sein. Insbesondere vorteilhaft ist es, wenn das wenigstens eine erste Aufnahmeelement zum formschlüssigen Halten des wenigstens einen Federelementes des Federbrücken-Atembeutel-Systems ausgebildet ist. Beispielsweise können Steckelemente, wie Zapfen oder Splinte, als Fixierelemente dienen.

Gemäß einem zweiten Aspekt der Erfindung wird die Aufgabe durch ein Federbrücken-Atembeutelplatte-System für ein Kreislaufatemschutzgerät, aufweisend eine Atembeutelplatte, eine Federbrücke sowie wenigstens ein Federelement, welches zwischen der Atembeutelplatte und der Federbrücke angeordnet ist, gelöst. Das Federbrücken-Atembeutelplatte-System ist dadurch gekennzeichnet, dass die Federbrücke gemäß dem zuvor beschriebenen ersten Aspekt der Erfindung, insbesondere gemäß den Ansprüchen 1 bis 8, ausgebildet ist. Ein derartig ausgebildetes Federbrücken-Atembeutelplatte-System für ein Kreislaufatemschutzgerät ermöglicht eine besonders einfache Bewegung des Federbrücken-Atembeutelplatte-Systems zwischen einer Arbeitsposition und einer Öffnungsposition und damit eine einfach Montage und Demontage eines Atembeutels von dem Federbrücken-Atembeutelplatte-System beziehungsweise aus dem Kreislaufatemschutzgerät. Dies wird dadurch gewährleistet, dass die Federbrücke und entsprechend das Federbrücken-Atembeutelplatte-System schwenk- und/oder schiebebeweglich an dem Gehäuse eines Kreislaufatemschutzgerätes angeordnet werden kann. Die Vorteile, die ausführlich bezüglich der Federbrücke gemäß dem ersten Aspekt der Erfindung beschrieben worden sind, gelten selbstverständlich auch für das Federbrücken-Atembeutelplatte-System gemäß dem zweiten Aspekt der Erfindung.

Dadurch, dass aufgrund des wenigstens einen Befestigungselementes der Federbrücke die Federbrücke relativ zum Gehäuse des Kreislaufatemschutzgerätes verschwenkt bzw. verschoben werden kann und gegebenenfalls von dem Gehäuse entfernt werden kann, ist ein besonders einfaches Entnehmen des Atembeutels des Kreislaufatemschutzgeräts aus dem Gehäuse des Kreislaufatemschutzgeräts gewährleistet. Die Atembeutelplatte ist lösbar an dem Atembeutel befestigt, sodass nach dem Verschwenken der Einheit, bestehend aus Federbrücke, wenigstens einem Federelement und Atembeutelplatte, der Atembeutel für einen Benutzer zur Demontage aus dem Kreislaufatemschutzgerät frei zugänglich ist. Das wenigstens eine Federelement des Federbrücken-Atembeutelplatte-Systems kann unterschiedlich ausgebildet sein. Beispielsweise kann das wenigstens eine Federelement als komprimierbares Elastomer oder Gummi ausgebildet sein. Besonders bevorzugt ist das wenigstens eine Federelement als Druckfeder oder Zugfeder ausgebildet. Vorzugsweise weist das Federbrücken-Atembeutelplatte-System zwei parallel zueinander verlaufende Druckfedern oder Zugfedern auf. Die jeweils ersten Enden der Druckfedern bzw. Zugfedern sind an dem Federbrückenträger form- und gegebenenfalls zusätzlich kraftschlüssig gehalten. Die jeweiligen zweiten Enden der Federelemente sind form- und auch hier gegebenenfalls zusätzlich kraftschlüssig an der Atembeutelplatte angeordnet. Vorzugsweise sind die Federelemente derartig ausgestaltet, dass diese eine Komprimierung in Richtung der Längsachse der Federelemente ermöglicht, aber ein Verschwenken der Federelemente relativ zur Längsachse der Federelemente möglichst unterbindet. Hierdurch ist eine gewisse Formstabilität der Federelemente sichergestellt. Auch eine andere Gestaltung der Druckfedern bzw. Zugfedern, welche hinsichtlich Ausknickung und geradliniger Kompressions- und Dekompressionswege optimiert sind, ist denkbar. Beispielsweise können die Federelemente als Druckfedern oder Zugfedern mit einem rechteckförmigen Federdrahtquerschnitt ausgebildet sein.

Federn dieser Bauart unterstützen geradlinige Kompression- und Dekompressionswege besonders. Die Atembeutelplatte ist lösbar an der Atembeuteloberseite des Atembeutels angeordnet. Dies kann beispielsweise durch Magnete, Druccknöpfe, Laschen oder formschlüssige Geometrien erfolgen.

Gemäß einer bevorzugten Weiterentwicklung der Erfindung kann bei einem Federbrücken-Atembeutelplatte-System vorgesehen sein, dass ein oder mehr Stabilisierungselemente zwischen der Atembeutelplatte und dem Federbrückenträger der Federbrücke angeordnet sind, zur definierten Auf- und Abbewegung der Atembeutelplatte und damit des Atembeutels in Richtung von und zur Federbrücke. D. h., das wenigstens eine Stabilisierungselement verhindert ein Ausknicken des wenigstens einen Federelementes, insbesondere der beiden Druckfedern, bei Kompression, also bei steigendem Atembeutelvolumen. Um ein Ausknicken des wenigstens einen Federelementes zu verhindern, ist die Atembeutelplatte bevorzugt mit zwei als Stabilisierungselemente ausgebildete Drahtbügeln linear und rotatorisch mit der Federbrücke verbunden. Durch die Stabilisierungselemente ist eine definierte Auf- und Abbewegung der Atembeutelplatte und damit des Atembeutels in allen Orientierungen des Kreislaufatemschutzgerätes ermöglicht. Das wenigstens eine Stabilisierungselement ist vorzugsweise derart ausgebildet, dass dieses nur eine lineare Bewegung beziehungsweise nur eine annähernd lineare Bewegung der Atembeutelplatte in Richtung des Federbrückenträgers bzw. aus der Richtung des Federbrückenträgers ermöglicht. Hierdurch kann gewährleistet werden, dass der an der Atembeutelplatte befestigte Atembeutel definiert komprimiert bzw. dekomprimiert wird. Das wenigstens eine Stabilisierungselement kann neben Drahtbügeln auch anders ausgebildet sein. Beispielsweise können ein oder mehrere Scherengelenke oder ein Führungsarm mit einem geometrisch außerhalb der Federbrücke liegenden Drehpunkt vorgesehen sein. Sowohl an der Federbrücke, d. h., insbesondere an dem Federbrückenträger, als auch an der Atembeutelplatte können entsprechende Aufnahmen für das wenigstens eine Stabilisierungselement vorgesehen sein. Diese Aufnahmen halten das wenigstens eine Stabilisierungselement sicher zwischen dem Federbrückenträger und der Atembeutelplatte, sodass die Funktionalität des wenigstens einen Stabilisierungselementes gewährleistet ist.

Bevorzugt kann bei einem Federbrücken-Atembeutelplatte-System vorgesehen sein, dass die Atembeutelplatte wenigstens ein zweites Aufnahmeelement zur Anordnung wenigstens eines zweiten Endes des wenigstens einen Federelementes des Federbrücken-Atembeutelplatte-Systems aufweist. Hierdurch ist sichergestellt, dass das wenigstens eine Federelement, vorzugsweise als Druckfedern oder Zugfedern ausgebildete Federelemente, sicher an der Atembeutelplatte angeordnet ist. Das wenigstens eine zweite Aufnahmeelement kann beispielsweise eine ringförmige Nut, ein rohr- oder hülsenförmiger Vorsprung oder ein Vorsprung in Form eines Topfes oder Zapfens an der Atembeutelplatte sein. Zusätzlich zum formschlüssigen Halten des wenigstens einen Federelementes an der Atembeutelplatte können Fixierelemente, beispielsweise in Form von kleinen Zapfen, vorgesehen sein, um einen besseren Halt, insbesondere kraftschlüssigen Halt, des wenigstens einen Federelementes an der Atembeutelplatte beziehungsweise dem wenigstens einen zweiten Aufnahmeelement der Atembeutelplatte zu gewährleisten.

Gemäß einem dritten Aspekt der Erfindung wird die Aufgabe durch ein Kreislaufatemschutzgerät, aufweisend ein Gehäuse mit einem Gehäuseunterteil und einem Gehäuseoberteil, einen Atembeutel, einem CO₂-Absorber, einer Sauerstoffflasche sowie ein Federbrücken-Atembeutelplatte-System gelöst. Erfindungsgemäß ist das Federbrücken-Atembeutelplatte-System des Kreislaufatemschutzgerätes gemäß dem zweiten Aspekt der Erfindung, insbesondere gemäß einem der Ansprüche 9 bis 12, ausgebildet. Ein derartiges Kreislaufatemschutzgerät weist die gleichen Vorteile auf, wie sie ausführlich gemäß der Federbrücke des ersten Aspekts der Erfindung bzw. des Federbrücken-Atembeutelplatte-Systems des zweiten Aspekts der Erfindung ausgeführt worden sind.

Ein derartig ausgebildetes Kreislaufatemschutzgerät kann nach einem Einsatz besonders einfach gewartet bzw. gereinigt werden. Insbesondere der Atembeutel des Kreislaufatemschutzgerätes ist aufgrund der Schwenk- und/oder Drehbeweglichkeit der Federbrücke des Federbrücken-Atembeutelplatte-Systems besonders einfach zugänglich. Nach einem Einsatz des Kreislaufatemschutzgeräts, beispielsweise bei einem Brand, kann die Federbrücke bzw. die Einheit aus Federbrücke, Federelement(en) und Atembeutelplatte, d.h. das Federbrücken-Atembeutelplatte-System, einfach aus einer Arbeitsposition in eine Öffnungsposition überführt werden und gegebenenfalls vollständig von dem Gehäuse des Kreislaufatemschutzgerätes entfernt werden, sodass ein Nutzer schnell und einfach zugänglich den Atembeutel aus dem Kreislaufatemschutzgerät entfernen kann, um diesen zu reinigen.

Bevorzugt kann bei einem Kreislaufatemschutzgerät vorgesehen sein, dass das Gehäuse, insbesondere das Gehäuseunterteil, wenigstens ein Gegenbefestigungselement zum dreh- und/oder schiebebeweglichen Halten der Federbrücke und damit des Federbrücken-Atembeutelplatte-Systems an dem Kreislaufatemschutzgerät mittels des wenigstens einen Befestigungselementes der Federbrücke aufweist. Das wenigstens eine Gegenbefestigungselement kann beispielsweise ein Zapfen sein, welcher an der Innenseite der Gehäusewandung des Gehäuseunterteils des Gehäuses des Kreislaufatemschutzgerätes hervorsteht. Entsprechende Befestigungselemente, insbesondere in Form von Steckaufnahmen, der Federbrücke ermöglichen sowohl ein Verschwenken der Federbrücke relativ zu dem Gehäuse des Kreislaufatemschutzgerätes sowie ein einfaches Entfernen der Federbrücke von dem Kreislaufatemschutzgerät. Hierdurch ist eine besonders schnelle und einfache Zugänglichkeit zu dem Atembeutel gewährleistet.

Gemäß einer besonders bevorzugten Weiterentwicklung der Erfindung kann bei einem Kreislaufatemschutzgerät vorgesehen sein, dass zwei gegenüberliegende Seiten des Gehäuses, insbesondere Seiten des Gehäuseunterteils, jeweils ein Gegenbefestigungselement zur Aufnahme von zwei Befestigungselementen des Federbrückenträgers aufweisen. Durch eine derartige Ausgestaltung des Kreislaufatemschutzgerätes ist eine besonders gute und sichere Anbindung der Federbrücke an das Gehäuse des Kreislaufatemschutzgerätes gewährleistet, insbesondere dient die Federbrücke beziehungsweise das Federbrücken-Atembeutelplatte-System somit auch als Stabilisierungselement innerhalb des Kreislaufatemschutzgerätes, da es zwei gegenüberliegende Wandungen des Gehäuseunterteils des Kreislaufatemschutzgerätes miteinander verbindet und somit als Stützelement unterhalb des Gehäuseoberteils verstärkend wirkt.

Gemäß einer weiteren Weiterentwicklung der Erfindung kann bei einem Federbrücken-Atembeutelplatte-System vorgesehen sein, dass der Atembeutel einen oder mehrere Vorsprünge aufweist, insbesondere weist der Atembeutel einen umlaufenden Kragen an der Atembeuteloberseite auf, an den bzw. in den die Atembeutelplatte formschlüssig aufgenommen werden kann. Hierdurch ist sichergestellt, dass die Atembeutelplatte in einer definierten Lage zu diesem angeordnet ist. Besonders bevorzugt ist die Atembeutelplatte durch Magnete, Druckknöpfe, Laschen oder formschlüssige Geometrien lösbar an der Oberseite des Atembeutels befestigt.

### BEVORZUGTE AUSFÜHRUNGSBEISPIELE

Weitere, die Erfindung verbessernde Maßnahmen ergeben sich aus der nachfolgenden Beschreibung zu Ausführungsbeispielen der Erfindung, welche in den Figuren dargestellt sind. Sämtliche aus den Ansprüchen, der Beschreibung und den Zeichnungen hervorgehende Merkmale und/oder Vorteile, einschließlich konstruktiver Einzelheiten und räumlicher Anordnungen, können sowohl für sich als auch in den verschiedenen Kombinationen erfindungswesentlich sein. Elemente mit gleicher Funktion und Wirkungsweise sind in den Zeichnungen mit denselben Bezugszeichen versehen. Es zeigen jeweils schematisch:
- Fig. 1: ein Federbrücken-Atembeutelplatte-System eines bekannten Kreislaufatemschutzgerätes in einer Schnittdarstellung,
- Fig. 2: in einer Seitenansicht ein erfindungsgemäßes Federbrücken-Atembeutelplatte-System für ein Kreislaufatemschutzgerät,
- Fig. 3: das Federbrücken-Atembeutelplatte-System gemäß Fig. 2 in einer Ansicht von unten,
- Fig. 4: eine perspektivische Ansicht des Federbrücken-Atembeutelplatte-Systems gemäß Fig. 2, wobei die Arretierungsvorrichtung der Federbrücke in einer Freigabeposition ist,
- Fig. 5: in einer perspektivischen Ansicht das Federbrücken-Atembeutelplatte-System gemäß Fig. 2, wobei die Arretierungsvorrichtung in der Arretierposition ist,
- Fig. 6: in einer perspektivischen Ansicht ein Federbrücken-Atembeutelplatte-System gemäß der Erfindung,
- Fig. 7: in einer Seitenansicht ein Atembeutel mit einer an dem Atembeutel angeordneten Atembeutelplatte eines Federbrücken-Atembeutelplatte-Systems,
- Fig. 8: das Federbrücken-Atembeutelplatte-System gemäß Fig. 6 in einer Seitenansicht,
- Fig. 9: in einer weiteren perspektivischen Ansicht das Federbrücken-Atembeutelplatte-System gemäß der Fig. 6,
- Fig. 10: in einer Explosionsdarstellung das Federbrücken-Atembeutelplatte-System gemäß der Fig. 6,
- Fig. 11: eine perspektivische Darstellung eines Gehäuseunterteils eines Kreislaufatemschutzgerätes mit einem erfindungsgemäßen Federbrücken-Atembeutelplatte-System in einer Arbeitsposition,
- Fig. 12: eine perspektivische Darstellung des Gehäuseunterteils gemäß Fig. 11, wobei das Federbrücken-Atembeutelplatte-System in eine Öffnungsposition verschwenkt ist,
- Fig. 13: eine weitere perspektivische Darstellung des Gehäuseunterteils gemäß Fig. 12, bei der das Federbrücken-Atembeutelplatte-System sich in einer Öffnungsposition befindet,
- Fig. 14: eine weitere perspektivische Darstellung des Gehäuseunterteils des Kreislaufatemschutzgerätes, wobei das Federbrücken-Atembeutelplatte-System sich in einer Arbeitsposition befindet,
- Fig. 15: eine Seitenansicht auf das Gehäuseunterteil des Kreislaufatemschutzgerätes gemäß Fig. 11, mit einem Federbrücken-Atembeutelplatte-System in Öffnungsposition,
- Fig. 16: in einer Seitenansicht das Gehäuseunterteil eines Kreislaufatemschutzgerätes gemäß Fig. 11 mit einem Federbrücken-Atembeutelplatte-System in einer Arbeitsposition, und
- Fig. 17: ein erfindungsgemäßes Kreislaufatemschutzgerät ohne Gehäuseoberteil.

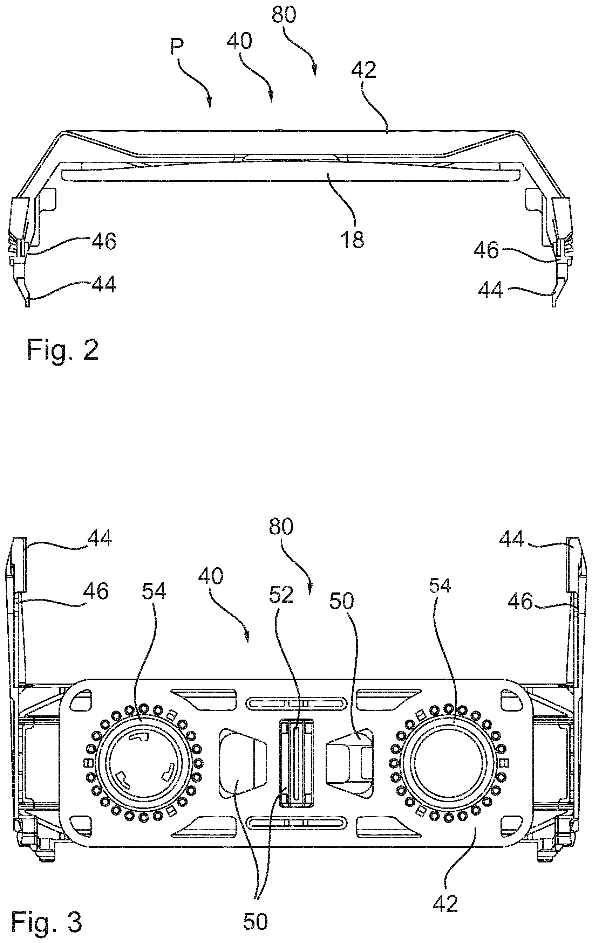

Fig. 2 zeigt schematisch in einer Seitenansicht ein erfindungsgemäßes Federbrücken-Atembeutelplatte-System 80 für ein Kreislaufatemschutzgerät. Fig. 3 zeigt schematisch das in Fig. 2 dargestellte Federbrücken-Atembeutelplatte-System 80 in einer Ansicht von unten. Das Federbrücken-Atembeutelplatte-System 80 weist eine Federbrücke 40 mit einem Federbrückenträger 42, zwei Federelemente 82 sowie eine Atembeutelplatte 18 auf. In der in Fig. 2 dargestellten Ansicht des Federbrücken-Atembeutelplatte-Systems 80 ist die Atembeutelplatte 18 maximal an den Federbrückenträger 42 der Federbrücke 40 herangeführt, wobei die beiden Federelemente 82 derart stark komprimiert sind, dass sie in der Fig. 2 nicht ersichtlich sind. In dieser Arretierposition P ist die Atembeutelplatte 18 an der Unterseite des Federbrückenträgers 42 mittels der nicht dargestellten Arretierungsvorrichtung 50 fixiert. Ein Atembeutel 10 eines Kreislaufatemschutzgerätes 100 könnte in der Arretierposition P von der Atembeutelplatte 18 leicht demontiert werden. Die Federbrücke 40 weist ein jochartiges bzw. U-förmig ausgebildetes Profil auf. An zwei sich gegenüberliegenden Seiten des Federbrückenträgers 42 sind jeweils Kragarme 46 angeordnet, die die Schenkel des U-förmigen Profils der Federbrücke 40 bilden. Die Kragarme 46 sind abgewinkelt zum Federbrückenträger 42 an dem Federbrückenträger 42 angeordnet. An den freien Enden der beiden Kragarme 46 ist jeweils ein Befestigungselement 44 in Form eines Drehelementes angeordnet. Das Drehelement 44 kann beispielsweise ein Drehgelenk, ein Kreuzgelenk, ein Kugelgelenk oder ein Scharnierelement sein. Bevorzugt ist das Drehelement 44 eine Steckaufnahme zur rotatorischen Befestigung an einem als Gegenbefestigungselement 108 ausgebildeten Zapfen an einer Gehäuseinnenwand eines Gehäuses 102 eines Kreislaufatemschutzgerätes 100. Durch derartig ausgebildete Befestigungselemente 44 kann die Federbrücke 40 einfach aus einer Arbeitsposition A in eine Öffnungsposition Ö verschwenkt werden. Vorzugsweise erfolgt eine Verschwenkung der Federbrücke 40 um 90° bzw. um annähernd 90° um die Drehachse der beiden Drehelemente 44. Hierdurch kann auf einfache und schnelle Art und Weise ein Zugang zu dem in der Arbeitsposition A unterhalb der Federbrücke 40 angeordneten Atembeutel 10 eines Kreislaufatemschutzgerätes 100 gewährleistet werden. Zu Reinigungszwecken kann der Atembeutel 10 dann sehr einfach aus dem Kreislaufatemschutzgerät 100 entfernt werden. Die Atembeutelplatte 18 des Federbrücken-Atembeutelplatte-Systems 80 weist erste Aufnahmeelemente 54 zur Aufnahme von Federelementen 82, die insbesondere als Druckfedern oder Zugfedern ausgebildet sind, auf. Die ersten Aufnahmeelement 54 sind vorzugsweise zum formschlüssigen Halten der Federelemente 82 des Federbrücken-Atembeutel-Systems 80 ausgebildet ist. Beispielsweise können Steckelemente, wie Zapfen oder Splinte, zusätzlich als Fixierelemente zum Fixieren der Federelemente 82 an den ersten Aufnahmeelementen 54 dienen.

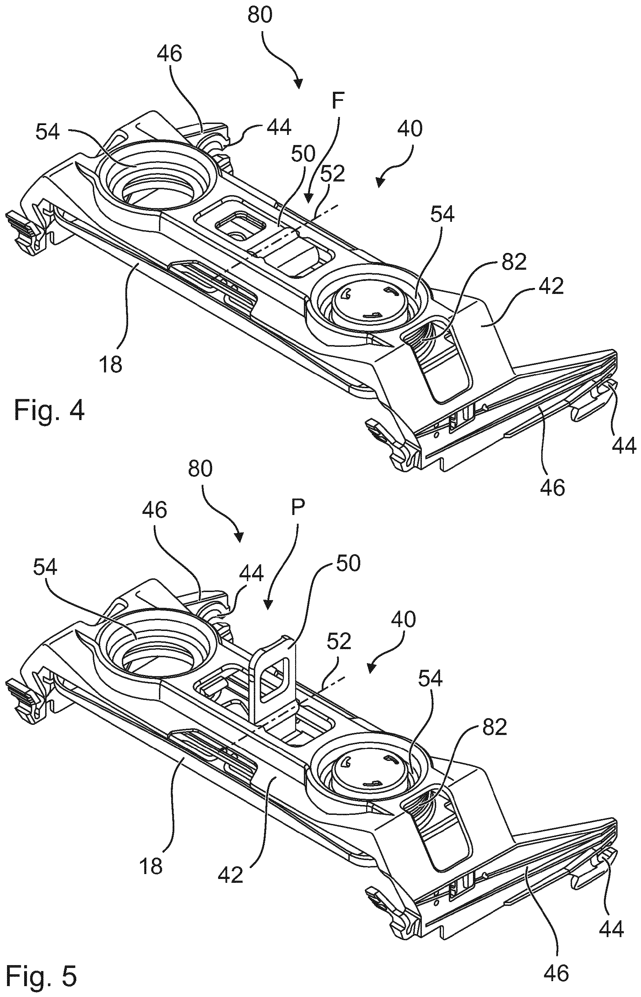

In den Fig. 4 und 5 ist das Federbrücken-Atembeutelplatte-System 80 gemäß den Fig. 2 und 3 in einer perspektivischen Ansicht dargestellt. Deutlich erkennbar ist in dieser Stellung, dass an dem freien Ende der Kragarme 46, d. h. an den Enden der Kragarme 46, welche dem Federbrückenträger 42 abgewandt sind, jeweils ein Befestigungselement 44 in Form einer rotatorischen Steckaufnahme angeordnet ist. Die Federbrücke 40 ist nicht mehr wie beim Stand der Technik an einer Gehäuseunterseite eines Gehäuses eines Kreislaufatemschutzgerätes fest vernietet, sondern über zwei an der Innenwandung der Gehäuseunterschale 104 hervorstehende Zapfen und die beiden steckbaren Steckaufnahmen 44 an den freien Enden der Kragarme 46 mit dem Gehäuse 102 eines Kreislaufatemschutzgerätes 100 miteinander rotatorisch verbindbar.

Die als Druckfedern ausgebildeten Federelemente 82 des Federbrücken-Atembeutelplatte-Systems 80 sind in den in den Fig. 4 und 5 dargestellten Abbildungen maximal komprimiert, sodass die Atembeutelplatte 18 direkt an der Unterseite des Federbrückenträgers 42 angeordnet ist. In der Fig. 4 befindet sich die an einer Schwenkachse 52 angeordnete Arretierungsvorrichtung 50 der Federbrücke 40 in einer Freigabeposition F. In dieser Freigabeposition F hält die Arretierungsvorrichtung 50 die Atembeutelplatte 18 nicht fest, sodass die Atembeutelplatte 18 aufgrund der Federkraft der Federelemente 82 von dem Federbrückenträger 42 weg bewegt werden könnte.

**In** der Fig. 5 befindet sich die Arretierungsvorrichtung 50 in einer Arretierposition P. In dieser Arretierposition P arretiert die Arretierungsvorrichtung 50 die Atembeutelplatte 18 an dem Federbrückenträger 42. D. h., die Arretierungsvorrichtung 50, die vorzugsweise als Rastelement oder Klemmelement ausgebildet ist, hintergreift beispielsweise eine entsprechende Hinterschneidung der Atembeutelplatte 18 und hält diese somit in einer definierten Position unterhalb des Federbrückenträgers 42 fest. In dieser Arretierposition P bildet das Federbrücken-Atembeutelplatte-System 80 eine sehr kompakte Einheit, welche einfach aus der Arbeitsposition A in eine Öffnungsposition Ö bewegt, insbesondere verschwenkt werden kann. Durch die kompakte Einheit des Federbrücken-Atembeutelplatte-Systems 80 sowie die Verschwenkung des Federbrücken-Atembeutelplatte-Systems 80 um die Schwenkachse des Drehelementes 44 entsteht viel Raum für den Zugang zu dem dann zugänglichen Atembeutel 10 eines Kreislaufatemschutzgerätes 100.

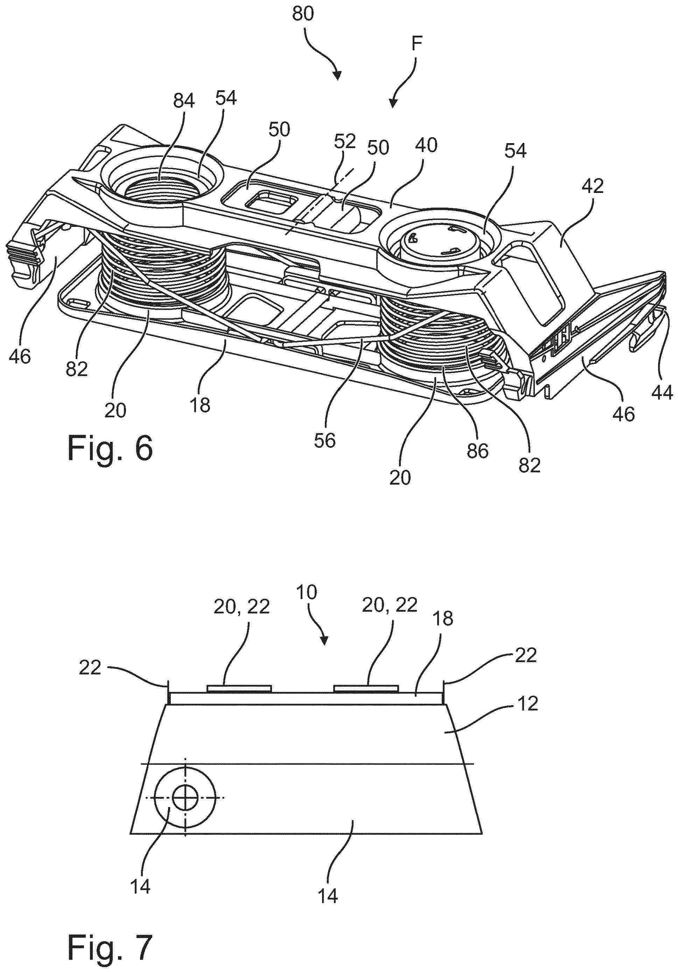

Fig. 6 zeigt schematisch das Federbrücken-Atembeutelplatte-System 80 gemäß denen in Fig. 2 bis 5 in einer perspektivischen Ansicht. Die um die Schwenkachse 52 schwenkbeweglich angeordnete Arretierungsvorrichtung 50 befindet sich in der Freigabeposition F, sodass die Atembeutelplatte 18 aufgrund der Federkraft der als Druckfedern ausgebildeten Federelemente 82 beabstandet zu dem Federbrückenträger 42 der Federbrücke 40 angeordnet ist. Das Federbrücken-Atembeutelplatte-System 80 weist zusätzlich ein Stabilisierungselement 56 auf, welches dafür sorgt, dass die Atembeutelplatte 18 geradlinig, d. h. parallel zu dem Federbrückenträger 42 der Federbrücke 40 verschoben wird, sowohl bei der Bewegung der Atembeutelplatte 18 in Richtung des Federbrückenträgers 42 als auch bei der Bewegung der Atembeutelplatte 18 von dem Federbrückenträger 42. Das Stabilisierungselement 56 sorgt dafür, dass die als Druckfedern ausgebildeten Federelemente 82 nicht abwinkeln, sondern geradlinig entlang ihrer Federachse sich komprimieren bzw. dekomprimieren können.

Fig. 7 zeigt schematisch in einer Seitenansicht einen Atembeutel 10 mit einer an dem Atembeutel 10 angeordneten Atembeutelplatte 18 eines Federbrücken-Atembeutelplatte-Systems 80. Die Atembeutelplatte 18 weist zwei zweite Aufnahmeelemente 20 auf zur formschlüssigen Aufnahme der jeweiligen zweiten Enden der Druck- oder Zugfedern 82 des Federbrücken-Atembeutelplatte-Systems 80. Die zweiten Aufnahmeelemente 20 sind vorzugsweise als ringförmige Nute, rohr-, topf- oder hülsenförmige Vorsprünge 22 ausgebildet. Zusätzlich zum formschlüssigen Halten der Druck- oder Zugfedern 82 an der Atembeutelplatte 18 können nicht dargestellte Fixierelemente, beispielsweise in Form von kleinen Zapfen, vorgesehen sein, um einen besseren Halt, insbesondere kraftschlüssigen Halt, der Druck- oder Zugfedern 82 an der Atembeutelplatte 18 beziehungsweise dem wenigstens einen zweiten Aufnahmeelement 20 der Atembeutelplatte 18 zu gewährleisten. Der Atembeutel 10 weist vorzugsweise eine stabile Unterseite 14 und eine zur stabilen Unterseite 14 bewegliche Oberseite 12 auf. Ferner können an der Unterseite 14 Anschlüsse, Muffen 16 angeordnet sein.

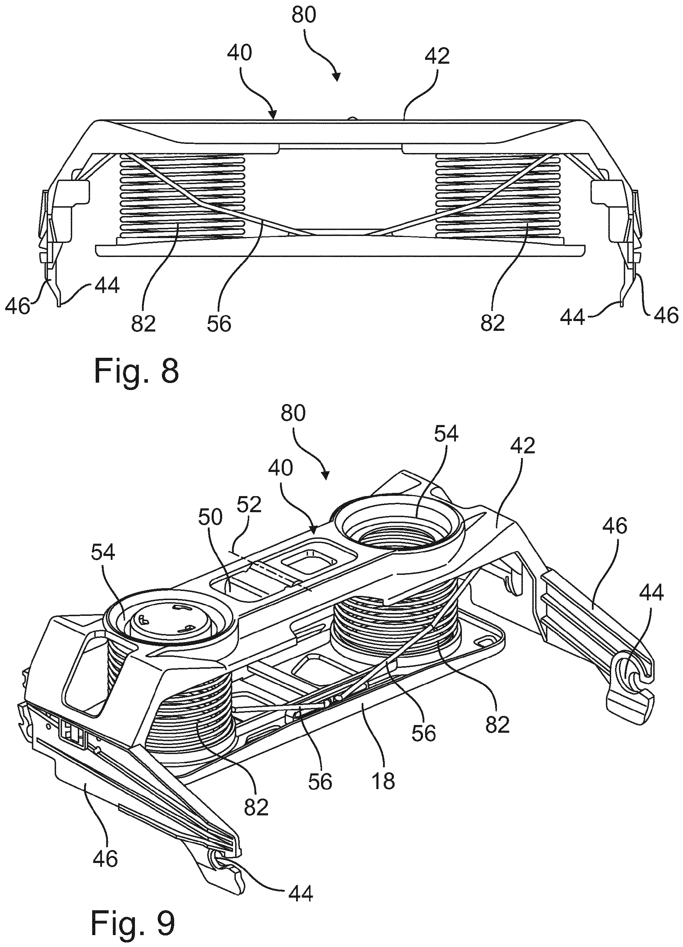

In Fig. 8 ist das Federbrücken-Atembeutelplatte-System 80 gemäß Fig. 6 in einer Seitenansicht, in Fig. 9 ist das Federbrücken-Atembeutelplatte-System 80 gemäß der Fig. 6 in einer weiteren perspektivischen Ansicht dargestellt. Deutlich erkennbar ist das Stabilisierungselement 56, welches dafür sorgt, dass die Atembeutelplatte 18 geradlinig, d. h. parallel zu dem Federbrückenträger 42 der Federbrücke 40 verschoben wird, bei einer Füllung beziehungsweise Leerung des Atembeutels 10 eines Kreislaufatemschutzgerätes 100. In Fig. 9 sind die Befestigungselement 44 an den Enden der Kragarme 46 gut erkennbar. Die Befestigungselement 44, hier Drehelemente, dienen zum einfach und definierten Verschwenken der Federbrücke 40 und damit des Federbrücken-Atembeutelplatte-Systems 80 relativ zum Gehäuse 102 eines Kreislaufatemschutzgerätes 100. Die hier dargestellten Drehelemente 44 sind sogenannte Steckaufnahmen die zur rotatorischen Drehung um entsprechende Gegenbefestigungselemente 108 an der Innenwandung eines Gehäuseunterteils 104 eines Gehäuses 102 eines Kreislaufatemschutzgerätes 100 auf diese Gegenbefestigungselemente 108 aussteckbar sind. Entsprechend sind die Steckaufnahmen auch wieder von den Gegenbefestigungselementen 108 entfernbar, sodass das Federbrücken-Atembeutelplatte-System 80 komplett aus dem Kreislaufatemschutzgerät 100 entnommen werden kann.

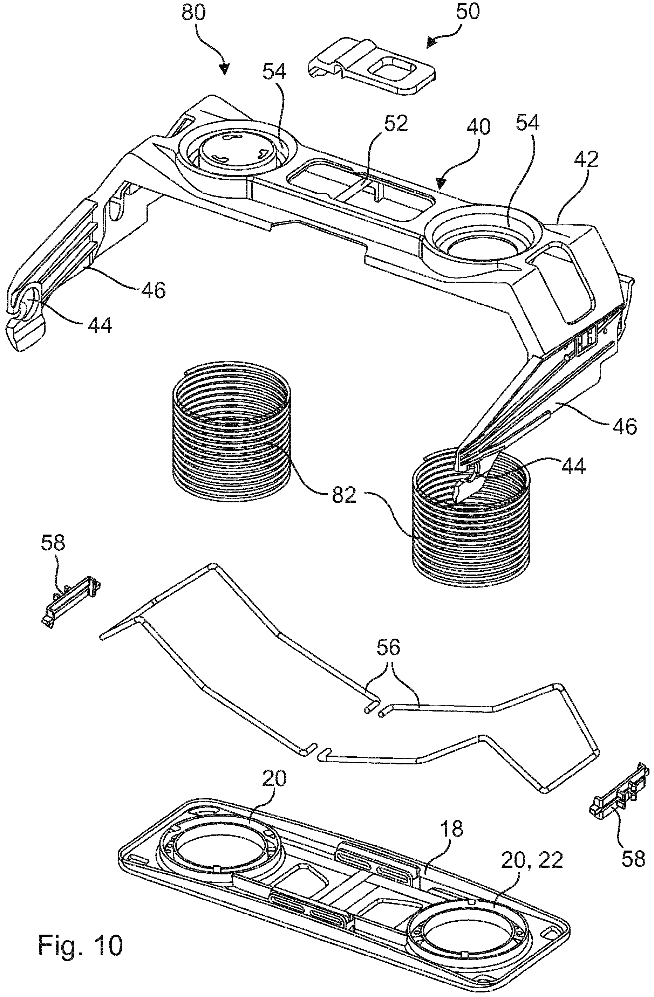

Fig. 10 zeigt schematisch in einer Explosionsdarstellung das Federbrücken-Atembeutelplatte-System 80 gemäß der Fig. 6. Die Arretierungsvorrichtung 50 ist als Schwenkhebel ausgebildet und kann an der Schwenkachse 52 des Federbrückenträgers 42 verschwenkbar gelagert werden. Die Federelemente 82 werden zwischen dem Federbrückenträger 42 und der Atembeutelplatte 18 angeordnet, vorzugsweise fixiert. Damit die Federelemente 82 geradlinig dekomprimiert und insbesondere komprimiert werden können, werden die Stabilisierungselement 56, hier in Form von Drahtbügeln, zwischen die Federbrückenträger 42 und die Atembeutelplatte 18 gespannt. Die Stabilisierungselemente 56 sorgt dafür, dass die als Druckfedern ausgebildeten Federelemente 82 nicht abwinkeln, sondern geradlinig entlang ihrer Federachse sich komprimieren bzw. dekomprimieren können. Entsprechend kann über die Atembeutelplatte 18 ein gleichmäßiger Druck auf den Atembeutel 10 eines Kreislaufatemschutzgeräts 100 ausgeübt werden.

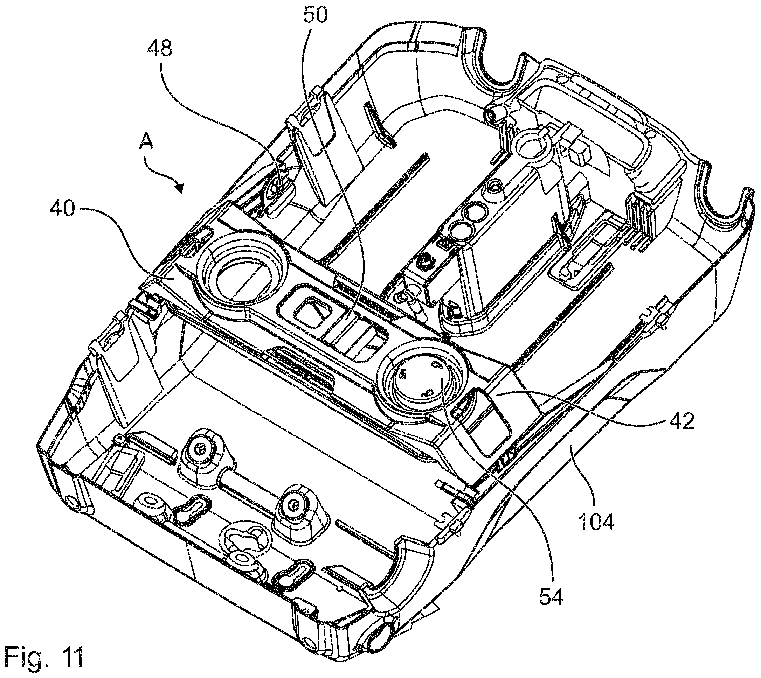

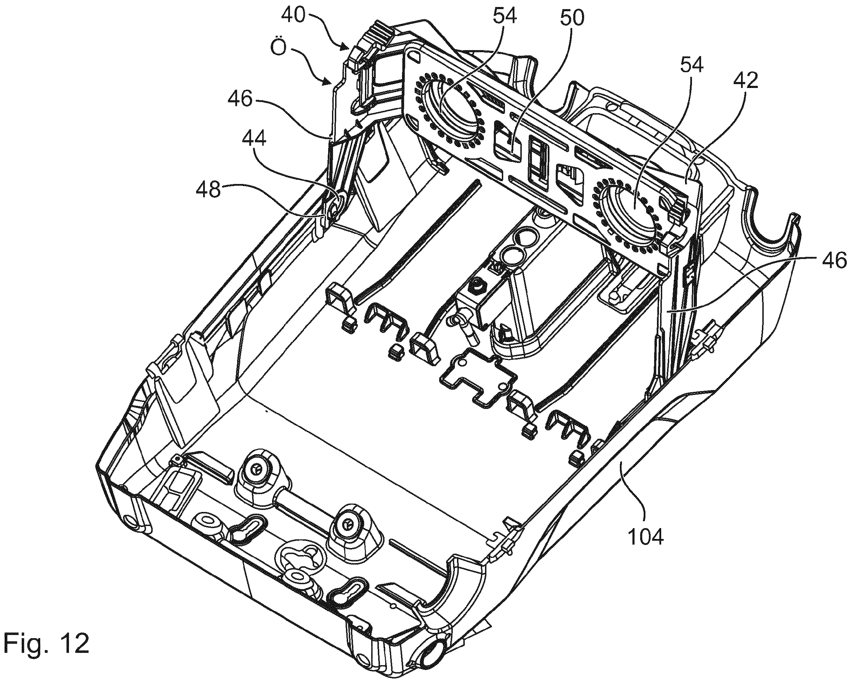

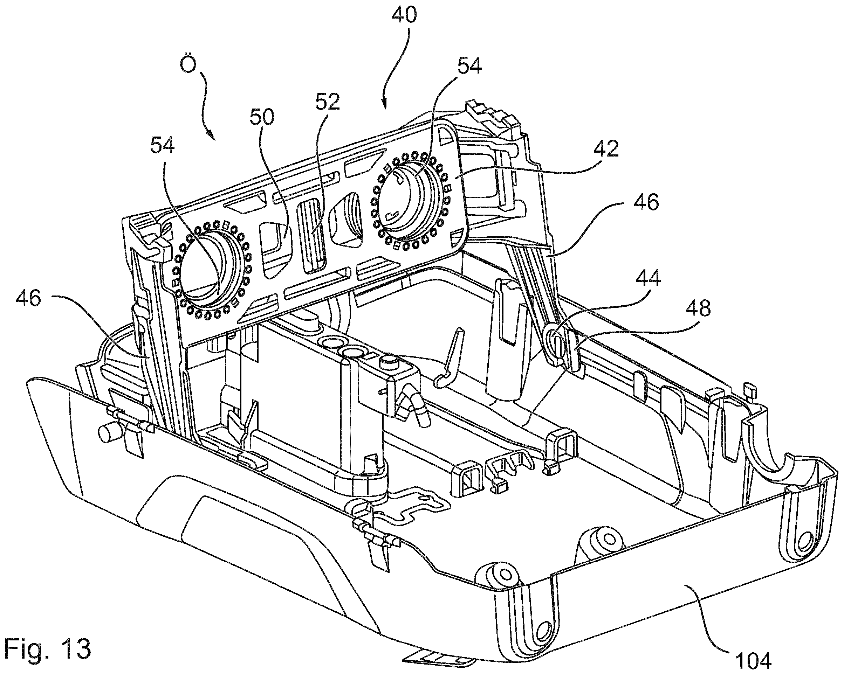

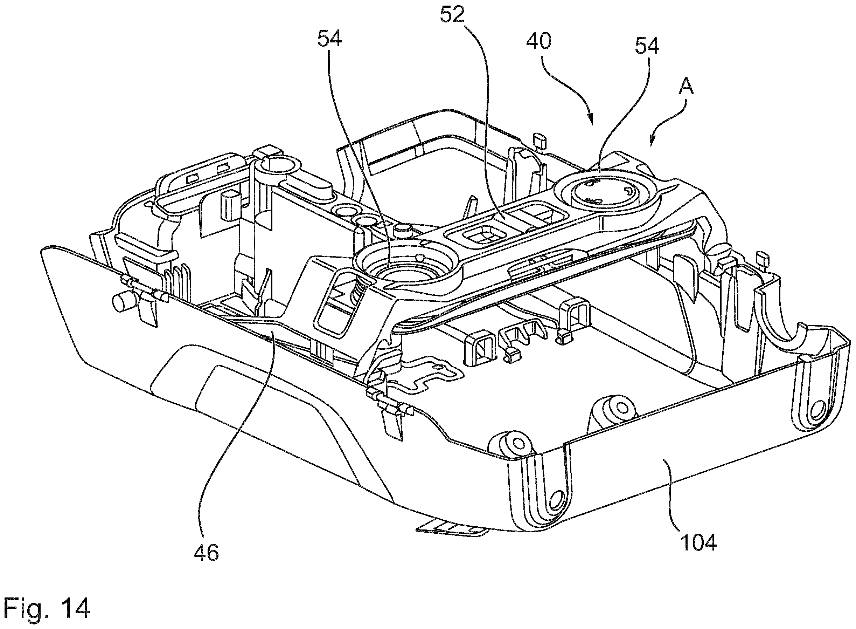

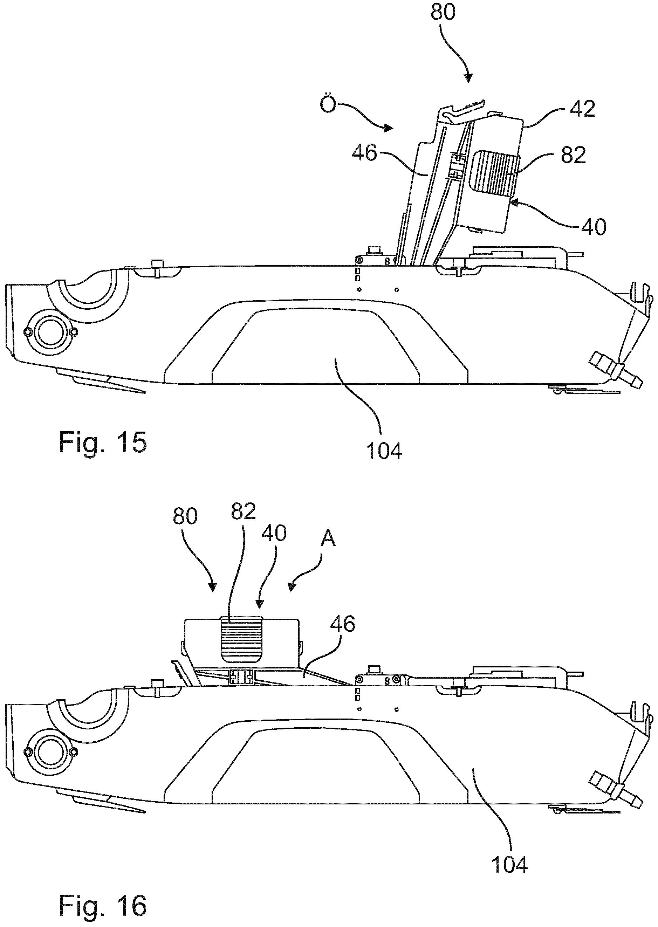

In den Fign. 11 bis 16 sind verschiedene Ansichten auf ein Gehäuseunterteil 104 eines Gehäuses 102 eines Kreislaufatemschutzgerätes 100 mit jeweils einem erfindungsgemäßen Federbrücken-Atembeutelplatte-System 80 dargestellt. In den Fign. 11, 14 und 16 befindet sich das Federbrücken-Atembeutelplatte-System 80 jeweils in einer Arbeitsposition A, in den Fig. 12, 13 und 15 befindet sich das Federbrücken-Atembeutelplatte-System 80 jeweils in einer Öffnungsposition Ö. In der Arbeitsposition A ist die Federbrücke 40 beziehungsweise das Federbrücken-Atembeutelplatte-System 80 derart an dem Gehäuseunterteil 104 des Kreislaufatemschutzgeräts 100 angeordnet, dass die Federelemente 82 die zwischen der Federbrücke 40 und dem Atembeutel 10 angeordnet sind, Druck auf den Atembeutel 10 ausüben können. D. h., in der Arbeitsposition A dient die Federbrücke 40 als Abstützung der Druckfedern beziehungsweise Zugfedern. In der Arbeitsposition A ist die Federbrücke 40 und damit das Federbrücken-Atembeutelplatte-System 80 unbeweglich, vorzugsweise verrastet, am Kreislaufatemschutzgerät 100 angeordnet. In der Öffnungsposition Ö sind die Federbrücke 40 und damit das Federbrücken-Atembeutelplatte-System 80 um 90° bzw um ungefähr 90° verschwenkt zum Vergleich zur Arbeitsposition A. Hierdurch wird der Atembeutel 10 eines Kreislaufatemschutzgerätes 100 freigegeben und kann einfach zur Reinigung ausgebaut werden.

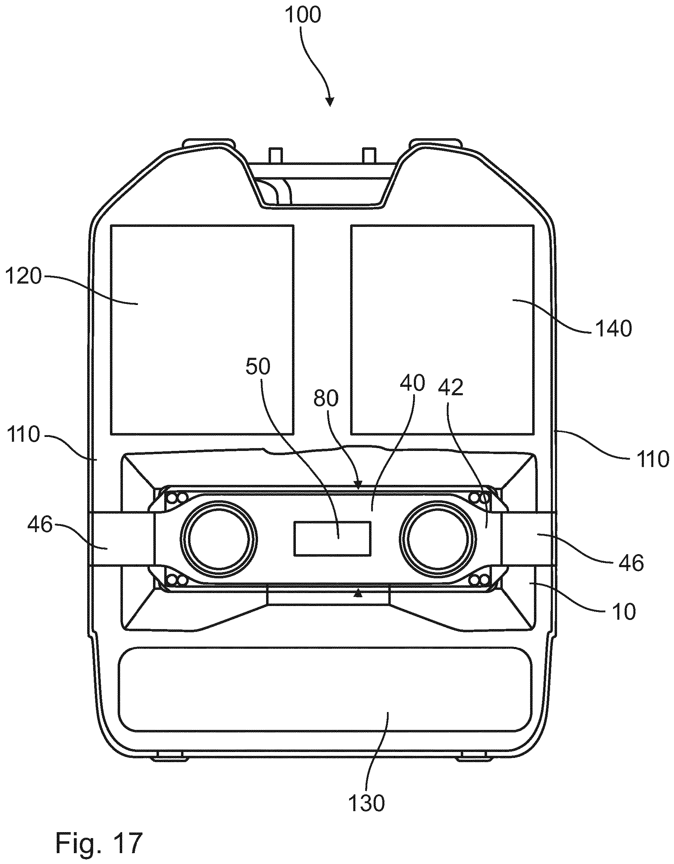

Die Federbrücke 40 und das Federbrücken-Atembeutelplatte-System 80 dienen zur Stabilisierung des Gehäuseunterteils 104 des Gehäuses 102 des Kreislaufatemschutzgerätes 100. Dies ist in Fig. 17 verdeutlicht. Dort ist schematisch ein erfindungsgemäßes Kreislaufatemschutzgerät 100 dargestellt. Das Kreislaufatemschutzgerät 100 weist ein Gehäuse 102 mit einem Gehäuseunterteil 104 und einem Gehäuseoberteil 106, einen CO₂-Absorber 120, einen Atembeutel 10, eine Sauerstoffflasche 130 sowie ein erfindungsgemäßes Federbrücken-Atembeutelplatte-System 80 auf. Über Befestigungselemente 44 an den freien Enden der Kragarme 46 ist das Federbrücken-Atembeutelplatte-System 80 dreh- und/oder schiebbeweglich an entsprechenden Gegenbefestigungselementen 108 an zwei gegenüberliegenden Innenwandungen des Gehäuseunterteils 104 rotatorisch befestigt.

### BEZUGSZEICHENLISTE

- 10: Atembeutel
- 12: Oberseite des Atembeutels
- 14: Unterseite des Atembeutels
- 16: Anschlüsse, Muffen
- 18: Druckplatte/Atembeutelplatte
- 20: zweites Aufnahmeelement
- 22: Vorsprung

- 40: Federbrücke
- 42: Federbrückenträger
- 44: Befestigungselement
- 46: Kragarm
- 48: Halteelement
- 50: Arretierungsvorrichtung
- 52: Schwenkachse
- 54: erstes Aufnahmeelement
- 56: Stabilisierungselement
- 58: Schnappelement

- 80: Federbrücken-Atembeutelplatte-System
- 82: Federelement
- 84: erstes Ende des Federelements
- 86: zweites Ende des Federelements

- 100: Kreislaufatemschutzgerät
- 102: Gehäuse
- 104: Gehäuseunterteil
- 106: Gehäuseoberteil
- 108: Gegenbefestigungselement
- 110: Seiten des Gehäuses
- 120: CO₂-Absorber
- 130: Sauerstoffflasche
- 140: Atemgaskühler-Einheit

- A: Arbeitsposition
- Ö: Öffnungsposition
- F: Freigabeposition
- P: Arretierposition

### Stand der Technik:

- 210: Atembeutel
- 212: Oberseite des Atembeutels
- 214: Unterseite des Atembeutels
- 216: Anschlüsse, Muffen
- 218: Druckplatte/Atembeutelplatte
- 240: Federbrücke
- 282: Druckfedern
- 1000: Kreislaufatemschutzgerät
- 1020: Gehäuse
- 1040: Gehäuseunterteil
- 1060: Gehäuseoberteil

## Patentansprüche

1. Federbrücke (40) für ein Federbrücken-Atembeutelplatte-System (80) eines Kreislaufatemschutzgeräts (100), aufweisend einen Federbrückenträger (42) zur Anordnung wenigstens eines Federelements (82) eines Federbrücken-Atembeutelplatte-Systems (80) sowie aufweisend zumindest ein Befestigungselement (44) zum Halten der Federbrücke (40) am Kreislaufatemschutzgerät (100),
wobei das zumindest eine Befestigungselement (44) ein Drehelement und/oder Schiebeelement zum dreh- und/oder schiebbeweglichen Bewegen der Federbrücke (40) am und relativ zum Kreislaufatemschutzgerät (100) ist, wobei die Federbrücke (40) mittels dem Drehelement und/oder dem Schiebeelement aus einer Arbeitsposition (A) in eine Öffnungsposition (Ö) drehbar und/oder verschiebbar ist zum Freigeben eines Zugangs zu einem Atembeutel (10) des Kreislaufatemschutzgeräts (100),
wobei die Federbrücke (40) in der Öffnungsposition (Ö) weiterhin am Kreislaufatemschutzgerät befestigt ist.

2. Federbrücke (40) nach Anspruch 1,
**dadurch gekennzeichnet, dass** das Drehelement ein Drehgelenk, ein Kreuzgelenk, ein Winkelelement, ein Kugelgelenk, ein Scharnierelement oder eine Steckaufnahme zur Befestigung an einem Zapfen des Kreislaufatemschutzgeräts (100) ist.

3. Federbrücke (40) nach Anspruch 1 oder 2,
**dadurch gekennzeichnet, dass** das zumindest eine Befestigungselement (44) an wenigstens einem Kragarm (46) der Federbrücke (40) angeordnet ist, wobei der wenigstens eine Kragarm (46) abgewinkelt zum Federbrückenträger (42) am dem Federbrückenträger (42) angeordnet ist.

4. Federbrücke (40) nach einem der vorangehenden Ansprüche,
**dadurch gekennzeichnet, dass** das Schiebeelement eine Führungskulisse oder ein Führungszapfen zur Führung der Federbrücke (40) an einem Gegenschiebeelement, insbesondere einem Gegenführungszapfen oder einer Gegenführungskulisse, des Kreislaufatemschutzgeräts (100) ist.

5. Federbrücke (40) nach einem der vorangehenden Ansprüche,
**dadurch gekennzeichnet, dass** die Federbrücke (40) ein Halteelement (48) zum Halten der Federbrücke (40) in der Arbeitsposition (A) und zum Halten der Federbrücke (40) in der Öffnungsposition (Ö) aufweist.

6. Federbrücke (40) nach einem der vorangehenden Ansprüche,
**dadurch gekennzeichnet, dass** der Federbrückenträger (42) eine Arretierungsvorrichtung (50) zum Arretieren einer durch das wenigstens eine Federelement (82) des Federbrücken-Atembeutelplatte-Systems (80) federkraftbeaufschlagten Atembeutelplatte (18) des Federbrücken-Atembeutelplatte-Systems (80) an dem Federbrückenträger (42) aufweist.

7. Federbrücke (1) nach Anspruch 6,
**dadurch gekennzeichnet, dass** die Arretierungsvorrichtung (50) über eine Schwenkachse (52) an dem Federbrückenträger (42) schwenkbeweglich angelenkt ist, insbesondere derart, dass in einer Freigabeposition (F), in der die Atembeutelplatte (18) des Federbrücken-Atembeutelplatte-Systems (80) nicht mittels der Arretierungsvorrichtung (50) an dem Federbrückenträger (42) gehalten ist, die Arretierungsvorrichtung (50) plan oder annähernd plan zu dem Federbrückenträger (42) verläuft, und in einer Arretierposition (P), in der die Atembeutelplatte (18) des Federbrücken-Atembeutelplatte-Systems (80) mittels der Arretierungsvorrichtung (50) an dem Federbrückenträger (42) gehalten ist, die Arretierungsvorrichtung (50) geneigt zu dem Federbrückenträger (42) verläuft.

8. Federbrücke (1) nach einem der vorangegangenen Ansprüche,
**dadurch gekennzeichnet, dass** der Federbrückenträger (42) wenigstens ein erstes Aufnahmeelement (54) zur Anordnung wenigstens eines ersten Endes (84) des wenigstens einen Federelements (82) des Federbrücken-Atembeutelplatte-Systems (80) aufweist.

9. Federbrücken-Atembeutelplatte-System (80) für ein Kreislaufatemschutzgerät (100), aufweisend eine Atembeutelplatte (18), eine Federbrücke (40) sowie wenigstens ein Federelement (82), welches zwischen der Atembeutelplatte (18) und der Federbrücke (40) angeordnet ist,
**dadurch gekennzeichnet, dass** die Federbrücke (40) nach einem der vorangegangenen Ansprüche ausgebildet ist.

10. Federbrücken-Atembeutelplatte-System (80) nach Anspruch 9,
**dadurch gekennzeichnet, dass** ein oder mehr Stabilisierungselemente (56) zwischen der Atembeutelplatte (18) und dem Federbrückenträger (42) der Federbrücke (40) angeordnet sind, zur definierten Auf- und Abbewegung der Atembeutelplatte (18) in Richtung von und zur Federbrücke.

11. Federbrücken-Atembeutelplatte-System (80) nach Anspruch 9 oder 10,
**dadurch gekennzeichnet, dass** die Atembeutelplatte (18) wenigstens ein zweites Aufnahmeelement (20) zur Anordnung wenigstens eines zweiten Endes (86) des wenigstens einen Federelements (82) des Federbrücken-Atembeutelplatte-Systems (80) aufweist.

12. Federbrücken-Atembeutelplatte-System (80) nach einem der vorangehenden Ansprüche 9 bis 11,
**dadurch gekennzeichnet, dass** die Atembeutelplatte (18) Befestigungsmittel aufweist zur lösbaren Befestigung der Atembeutelplatte (18) an der Atembeuteloberseite (12) eines Atembeutels (10).

13. Kreislaufatemschutzgerät (100), aufweisend ein Gehäuse (102) mit einem Gehäuseunterteil (104) und einem Gehäuseoberteil (106), einen CO₂-Absorber (120), einen Atembeutel (10), eine Sauerstoffflasche (130) sowie ein Federbrücken-Atembeutelplatte-System (80),
**dadurch gekennzeichnet, dass** das Federbrücken-Atembeutelplatte-System (80) nach einem der vorangegangenen Ansprüche 9 bis 12 ausgebildet ist.

14. Kreislaufatemschutzgerät (100) nach Anspruch 13,
**dadurch gekennzeichnet, dass** das Gehäuse, insbesondere das Gehäuseunterteil (104), wenigstens ein Gegenbefestigungselement (108) zum dreh- und/oder schiebbeweglichen Halten der Federbrücke (40) an dem Kreislaufatemschutzgerät (100) mittels des wenigstens einen Befestigungselements (44) des Federbrückenträgers (42) aufweist.

15. Kreislaufatemschutzgerät (100) nach Anspruch 13 oder 14,
**dadurch gekennzeichnet, dass** an zwei gegenüberliegenden Seiten (110) des Gehäuses (102), insbesondere des Gehäuseunterteils (104), jeweils ein Gegenbefestigungselement (108) zur Aufnahme von zwei Befestigungselementen (44) des Federbrückenträgers (42) vorgesehen ist.

16. Kreislaufatemschutzgerät (100) nach einem der vorangehenden Ansprüche 13 bis 15, **dadurch gekennzeichnet, dass** der Atembeutel (10) einen oder mehrere Vorsprünge (22) zum formschlüssigen Aufnehmen der Atembeutelplatte (18) aufweist.

## Claims

1. Spring bridge (40) for a spring bridge breathing bag plate system (80) of a closed-circuit breathing apparatus (100), comprising a spring bridge carrier (42) for arranging at least one spring element (82) of a spring bridge breathing bag plate system (80) and comprising at least one fastening element (44) for holding the spring bridge (40) on the closed-circuit breathing apparatus (100),
wherein the at least one fastening element (44) is a rotary element and/or sliding element for rotatably and/or slidably moving the spring bridge (40) on and relative to the closed-circuit breathing apparatus (100), wherein the spring bridge (40) is rotatable and/or slidable by means of the rotary element and/or the sliding element from a working position (A) into an opening position (Ö) to release access to a breathing bag (10) of the closed-circuit breathing apparatus (100),
wherein the spring bridge (40) remains fastened to the closed-circuit breathing apparatus when the spring bridge is in the opening position (Ö).

2. Spring bridge (40) according to claim 1,
**characterized in that** the rotary element is a rotary joint, a universal joint, an angle element, a ball joint, a hinge element or a plug-in receptacle for fastening to a pin of the closed-circuit breathing apparatus (100).

3. Spring bridge (40) according to either claim 1 or claim 2,
**characterized in that** the at least one fastening element (44) is arranged on at least one cantilever arm (46) of the spring bridge (40), the at least one cantilever arm (46) being arranged on the spring bridge carrier (42) at an angle to the spring bridge carrier (42).

4. Spring bridge (40) according to any of the preceding claims,
**characterized in that** the sliding element is a guide slot or a guide pin for guiding the spring bridge (40) on a counter-sliding element, in particular a counter-guide pin or a counter-guide slot, of the closed-circuit breathing apparatus (100).

5. Spring bridge (40) according to any of the preceding claims,
**characterized in that** the spring bridge (40) has a holding element (48) for holding the spring bridge (40) in the working position (A) and for holding the spring bridge (40) in the opening position (Ö).

6. Spring bridge (40) according to any of the preceding claims,
**characterized in that** the spring bridge carrier (42) has a locking device (50) for locking a breathing bag plate (18) of the spring bridge breathing bag plate system (80), which breathing bag plate is spring-loaded by the at least one spring element (82) of the spring bridge breathing bag plate system (80), to the spring bridge carrier (42).

7. Spring bridge (1) according to claim 6,
**characterized in that** the locking device (50) is pivotally hinged to the spring bridge carrier (42) via a pivot axis (52), in particular such that, in a release position (F), in which the breathing bag plate (18) of the spring bridge breathing bag plate system (80) is not held on the spring bridge carrier (42) by means of the locking device (50), the locking device (50) runs flat or approximately flat to the spring bridge carrier (42), and, in a locking position (P), in which the breathing bag plate (18) of the spring bridge breathing bag plate system (80) is held on the spring bridge carrier (42) by means of the locking device (50), the locking device (50) runs inclined to the spring bridge carrier (42).

8. Spring bridge (1) according to any of the preceding claims,
**characterized in that** the spring bridge carrier (42) has at least one first receiving element (54) for arranging at least one first end (84) of the at least one spring element (82) of the spring bridge breathing bag plate system (80).

9. Spring bridge breathing bag plate system (80) for a closed-circuit breathing apparatus (100), comprising a breathing bag plate (18), a spring bridge (40) and at least one spring element (82) which is arranged between the breathing bag plate (18) and the spring bridge (40),
**characterized in that** the spring bridge (40) is designed according to any of the preceding claims.

10. Spring bridge breathing bag plate system (80) according to claim 9,
**characterized in that** one or more stabilizing elements (56) are arranged between the breathing bag plate (18) and the spring bridge carrier (42) of the spring bridge (40), for the defined up and down movement of the breathing bag plate (18) in the direction from and towards the spring bridge.

11. Spring bridge breathing bag plate system (80) according to claim 9 or 10,
**characterized in that** the breathing bag plate (18) has at least one second receiving element (20) for arranging at least one second end (86) of the at least one spring element (82) of the spring bridge breathing bag plate system (80).

12. Spring bridge breathing bag plate system (80) according to any of the preceding claims 9 to 11,
**characterized in that** the breathing bag plate (18) has fastening means for releasably fastening the breathing bag plate (18) to the breathing bag top side (12) of a breathing bag (10).

13. Closed-circuit breathing apparatus (100), comprising a housing (102) that has a lower housing part (104) and an upper housing part (106), a CO₂ absorber (120), a breathing bag (10), an oxygen bottle (130) and a spring bridge breathing bag plate system (80),
**characterized in that** the spring bridge breathing bag plate system (80) is designed according to any of the preceding claims 9 to 12.

14. Closed-circuit breathing apparatus (100) according to claim 13,
**characterized in that** the housing, in particular the lower housing part (104), has at least one counter-fastening element (108) for rotatably and/or slidably holding the spring bridge (40) on the closed-circuit breathing apparatus (100) by means of the at least one fastening element (44) of the spring bridge carrier (42).

15. Closed-circuit breathing apparatus (100) according to either claim 13 or claim 14,
**characterized in that** on each of two opposite sides (110) of the housing (102), in particular of the lower housing part (104), a counter-fastening element (108) is provided for receiving two fastening elements (44) of the spring bridge carrier (42).

16. Closed-circuit breathing apparatus (100) according to any of the preceding claims 13 to 15, **characterized in that** the breathing bag (10) comprises one or more projections (22) for form-fittingly receiving the breathing bag plate (18).

## Revendications

1. Couvercle à ressort (40) pour un système à plaque de sac respiratoire à couvercle à ressort (80) d'un appareil de protection respiratoire à circuit fermé (100), présentant un support de couvercle à ressort (42) pour la disposition d'au moins un élément formant ressort (82) d'un système à plaque de sac respiratoire à couvercle à ressort (80) ainsi que présentant au moins un élément de fixation (44) pour le maintien du couvercle à ressort (40) sur l'appareil de protection respiratoire à circuit fermé (100),
dans lequel l'au moins un élément de fixation (44) est un élément rotatif et/ou un élément coulissant pour le déplacement rotatif et/ou coulissant du couvercle à ressort (40) sur l'appareil de protection respiratoire à circuit fermé (100) ou par rapport à celui-ci, dans lequel le couvercle à ressort (40) peut être tourné et/ou coulissé au moyen de l'élément rotatif et/ou de l'élément coulissant d'une position de travail (A) à une position d'ouverture (Ö) pour la libération d'un accès à un sac respiratoire (10) de l'appareil de protection respiratoire à circuit fermé (100),
dans lequel le couvercle à ressort (40) est en outre fixé à l'appareil de protection respiratoire à circuit fermé dans la position d'ouverture (Ö).

2. Couvercle à ressort (40) selon la revendication 1,
**caractérisé en ce que** l'élément rotatif est une charnière, un joint universel, un élément angulaire, un joint à rotule, un élément formant charnière ou un logement enfichable permettant la fixation à un pivot de l'appareil de protection respiratoire à circuit fermé (100).

3. Couvercle à ressort (40) selon la revendication 1 ou 2,
**caractérisé en ce que** l'au moins un élément de fixation (44) est disposé sur au moins un bras en porte-à-faux (46) du couvercle à ressort (40), dans lequel l'au moins un bras en porte-à-faux (46) est disposé sur le support de couvercle à ressort (42) de manière coudée par rapport au support de couvercle à ressort (42).

4. Couvercle à ressort (40) selon l'une des revendications précédentes,
**caractérisé en ce que** l'élément coulissant est une coulisse de guidage ou un pivot de guidage pour le guidage du couvercle à ressort (40) sur un contre-élément coulissant, en particulier un contre-pivot de guidage ou une contre-coulisse de guidage, de l'appareil de protection respiratoire à circuit fermé (100).

5. Couvercle à ressort (40) selon l'une des revendications précédentes,
**caractérisé en ce que** le couvercle à ressort (40) présente un élément de maintien (48) pour le maintien du couvercle à ressort (40) dans la position de travail (A) et pour le maintien du couvercle à ressort (40) dans la position d'ouverture (Ö).

6. Couvercle à ressort (40) selon l'une des revendications précédentes,
**caractérisé en ce que** le support de couvercle à ressort (42) présente un dispositif de blocage (50) pour le blocage d'une plaque de sac respiratoire (18) du système à plaque de sac respiratoire à couvercle à ressort (80) sur le support de couvercle à ressort (42), laquelle plaque de sac respiratoire est sollicitée par une force de ressort de l'au moins un élément formant ressort (82) du système à plaque de sac respiratoire à couvercle à ressort (80).

7. Couvercle à ressort (1) selon la revendication 6,
**caractérisé en ce que** le dispositif de blocage (50) est articulé de manière pivotante sur le support de couvercle à ressort (42) par l'intermédiaire d'un axe de pivotement (52), en particulier de telle sorte que, dans une position de libération (F) dans laquelle la plaque de sac respiratoire (18) du système à plaque de sac respiratoire à couvercle à ressort (80) n'est pas maintenue sur le support de couvercle à ressort (42) au moyen du dispositif de blocage (50), le dispositif de blocage (50) s'étend de manière plane ou approximativement plane par rapport au support de couvercle à ressort (42) et, dans une position de blocage (P) dans laquelle la plaque de sac respiratoire (18) du système à plaque de sac respiratoire à couvercle à ressort (80) est maintenue sur le support de couvercle à ressort (42) au moyen du dispositif de blocage (50), le dispositif de blocage (50) s'étend de manière inclinée par rapport au support de couvercle à ressort (42).

8. Couvercle à ressort (1) selon l'une des revendications précédentes,
**caractérisé en ce que** le support de couvercle à ressort (42) présente au moins un premier élément de réception (54) pour la disposition d'au moins une première extrémité (84) de l'au moins un élément formant ressort (82) du système à plaque de sac respiratoire à couvercle à ressort (80).

9. Système à plaque de sac respiratoire à couvercle à ressort (80) pour un appareil de protection respiratoire à circuit fermé (100), présentant une plaque de sac respiratoire (18), un couvercle à ressort (40) ainsi qu'au moins un élément formant ressort (82) qui est disposé entre la plaque de sac respiratoire (18) et le couvercle à ressort (40),
**caractérisé en ce que** le couvercle à ressort (40) est réalisé selon l'une des revendications précédentes.

10. Système à plaque de sac respiratoire à couvercle à ressort (80) selon la revendication 9,
**caractérisé en ce qu'**un ou plusieurs éléments de stabilisation (56) sont disposés entre la plaque de sac respiratoire (18) et le support de couvercle à ressort (42) du couvercle à ressort (40) pour le déplacement défini vers le haut et vers le bas de la plaque de sac respiratoire (18) en direction et en provenance du couvercle à ressort.

11. Système à plaque de sac respiratoire à couvercle à ressort (80) selon la revendication 9 ou 10,
**caractérisé en ce que** la plaque de sac respiratoire (18) présente au moins un second élément de réception (20) pour la disposition d'au moins une seconde extrémité (86) de l'au moins un élément formant ressort (82) du système à plaque de sac respiratoire à couvercle à ressort (80).

12. Système à plaque de sac respiratoire à couvercle à ressort (80) selon l'une des revendications précédentes 9 à 11,
**caractérisé en ce que** la plaque de sac respiratoire (18) présente un moyen de fixation pour la fixation de manière amovible de la plaque de sac respiratoire (18) sur la face supérieure de sac respiratoire (12) d'un sac respiratoire (10).

13. Appareil de protection respiratoire à circuit fermé (100), présentant un boîtier (102) comportant une partie inférieure de boîtier (104) et une partie supérieure de boîtier (106), un absorbeur de CO₂ (120), un sac respiratoire (10), une bouteille d'oxygène (130) ainsi qu'un système à plaque de sac respiratoire à couvercle à ressort (80),
**caractérisé en ce que** le système à plaque de sac respiratoire à couvercle à ressort (80) est réalisé selon l'une des revendications précédentes 9 à 12.

14. Appareil de protection respiratoire à circuit fermé (100) selon la revendication 13,
**caractérisé en ce que** le boîtier, en particulier la partie inférieure de boîtier (104), présente au moins un contre-élément de fixation (108) pour le maintien en déplacement rotatif et/ou coulissant du couvercle à ressort (40) sur l'appareil de protection respiratoire à circuit fermé (100) au moyen de l'au moins un élément de fixation (44) du support de couvercle à ressort (42).

15. Appareil de protection respiratoire à circuit fermé (100) selon la revendication 13 ou 14,
**caractérisé en ce que,** sur deux faces opposées (110) du boîtier (102), en particulier de la partie inférieure de boîtier (104), respectivement un contre-élément de fixation (108) est prévu pour la réception de deux éléments de fixation (44) du support de couvercle à ressort (42).

16. Appareil de protection respiratoire à circuit fermé (100) selon l'une des revendications précédentes 13 à 15, **caractérisé en ce que** le sac respiratoire (10) présente une ou plusieurs saillies (22) pour la réception par complémentarité de forme de la plaque de sac respiratoire (18).
